# FASCICULE DE BREVET EUROPEEN

(11) **EP 3 916 380 B1**
(45) Date de publication et mention de la délivrance du brevet: **31.01.2024**
(21) Numéro de dépôt: 21186684.3
(22) Date de dépôt: 06.12.2018
(51) Int. Cl.: G01N 23/046, G07C 3/14

(54) **MÉTHODE ET MACHINE POUR CONTRÔLER UN PROCÉDÉ DE FORMAGE**
VERFAHREN UND MASCHINE ZUR STEUERUNG EINES FORMVERFAHRENS
METHOD AND MACHINE FOR CONTROLLING A FORMING METHOD

(30) Priorité: 08.12.2017 FR 1761865
(43) Date de publication de la demande: 01.12.2021
(62) Demande divisionnaire de: 18833452.8
(73) Titulaire: TIAMA, 69230 Saint-Genis-Laval (FR)
(72) Inventeur: COSNEAU, Laurent, 69510 Soucieu-en-Jarrest (FR); COLLE, Olivier, 69600 Oullins (FR)
(74) Mandataire: Cabinet Beau de Loménie

(56) Documents cités:
- WO-A1-2013/185816
- GB-A- 1 432 120
- GB-A- 2 094 530
- Anthon Du Plessis: "CT News: The Stellenbosch CT scanner facility newsletter, vol. 2, no 1", , 1 janvier 2014 (2014-01-01), XP055339995, Extrait de l'Internet: URL:http://blogs.sun.ac.za/ctscanner/files /2013/07/CT-News-2014-Issue-1_final1.pdf [extrait le 2017-01-27]

## Description

La présente invention concerne le domaine technique de la fabrication de récipients en verre tels que par exemple des bouteilles, des pots ou des flacons, mettant en oeuvre une installation de formage comportant plusieurs sections de formage distinctes.

De manière connue, une installation de formage comporte plusieurs sections de formage comprenant chacune au moins un moule ébaucheur et au moins un moule finisseur. Cette installation comporte également un distributeur de paraisons de verre fondu ou gouttes de verre malléable tombant par gravité dans chaque moule ébaucheur. Une paraison de verre fondu est en premier lieu mise en forme d'une ébauche dans le moule ébaucheur, puis est transférée pour une mise en forme finale dans le moule finisseur. Chaque récipient extrait du moule finisseur à une température encore de l'ordre de 600°C est acheminé vers différents postes de traitement et de contrôle.

Le contrôle de la qualité des récipients en verre est nécessaire afin d'éliminer ceux qui présentent des défauts susceptibles d'affecter leur caractère esthétique ou plus grave, de constituer un réel danger pour l'utilisateur ultérieur.

Un premier critère de qualité des récipients en verre concerne la répartition du verre c'est-à-dire la distribution de l'épaisseur de la paroi en verre.

La répartition de verre dans les récipients fabriqués dépend de plusieurs paramètres de contrôle du procédé de formage, comme la qualité du chargement du verre dans le moule ébaucheur. En effet, le centrage de la goutte de verre par rapport au moule ébaucheur, le timing d'arrivée de la goutte, son orientation/inclinaison lors de son entrée dans le moule influent directement sur la répartition de verre des récipients produits. D'autres caractéristiques influent également sur cette répartition, par exemple la lubrification et la ventilation des moules, la distribution de la température dans la goutte, les déformations de la goutte durant la distribution.

Par ailleurs, les moules et notamment le moule finisseur déterminent la forme des récipients, plus précisément sa surface extérieure. Le formage de la goutte de verre détermine la quantité de verre constituant le récipient. Cependant, la surface interne du récipient est produite par perçage ou soufflage de la goutte de verre chargée dans le moule ébaucheur, puis l'ébauche obtenue est soufflée dans le moule finisseur. Aussi la surface interne dépend de nombreux paramètres de contrôle du procédé, et l'épaisseur peut varier selon ces paramètres en différents endroits du récipient final. Par exemple, la paroi verticale du corps peut présenter des régions plus épaisses ou plus minces, une partie du fond peut être plus épaisse, par exemple l'intérieur du fond peut être inclinée ou en trapèze au lieu d'être plate. Il est possible que la partie de l'épaule correspondant au demi-moule droit soit plus épaisse que la partie opposée. Dans une autre situation, l'épaisseur du bas du corps peut augmenter au détriment du haut du corps. Il arrive également que des zones minces inférieures à un seuil déterminé apparaissent au niveau du talon ou de l'épaule du récipient.

Une répartition anormale du verre est un défaut de fabrication qu'il faut corriger. Il est à considérer qu'il apparaît intéressant d'identifier un défaut de formage le plus tôt possible à la sortie de l'installation de formage de manière à le corriger le plus tôt possible au niveau de cette installation. Dans l'état de la technique, diverses solutions ont été proposées pour contrôler la répartition de verre des récipients à haute température sortant de la machine de formage.

Une méthode simple mais peu précise est l'observation manuelle par les opérateurs qui sectionnent un récipient et observent l'épaisseur de la paroi au niveau de la section. Eventuellement un pied à coulisse, un palpeur ou un gabarit peuvent donner une valeur de mesure. Cette méthode destructive, utilisée avec parcimonie donne une mesure peu précise et limitée à l'emplacement de la coupe.

Des capteurs manuels d'épaisseur de verre existent. Par exemple les capteurs manuels à effet hall mesurent la distance entre une bille intérieure et un capteur en contact avec la face externe. Ils sont précis mais uniquement manuels et il faut beaucoup de temps pour obtenir une répartition de verre sur l'ensemble du récipient. De plus, cette mesure ne permet pas de guider les opérateurs dans le pilotage du procédé de formage.

Une autre méthode est l'observation des récipients chauds en défilement sur le convoyeur de sortie, par une caméra infra-rouge en partant du principe que les régions épaisses des récipients rayonnent davantage. En conséquence, l'analyse des images infra-rouges des récipients dans des parties différentes, traduit probablement des répartitions hétérogènes de verre. Cependant, des défauts de répartition des températures causant également des hétérogénéités de rayonnement, ni l'opérateur ni la machine d'inspection ne disposent d'une information réelle de la répartition du verre. De plus, des régions sont cachées à la caméra, même si deux caméras sont utilisées.

Un autre critère de qualité des récipients en verre concerne la capacité nominale ou totale des récipients.

La capacité ou la contenance d'un récipient est le volume de liquide minimal qu'il contient si il est rempli à ras bord ou jusqu'à une hauteur déterminée sous la surface de la bague du récipient. Des dispositions réglementaires ou administratives imposent de connaître avec précision la capacité des récipients. La capacité réelle des récipients doit correspondre à la capacité indiquée sur le récipient qui est par exemple gravée sur le récipient ou inscrite sur une étiquette rapportée sur le récipient.

Certaines dérives du procédé de fabrication des récipients peuvent conduire à des variations de leur capacité. A volume de verre constant, si le volume du moule finisseur augmente par usure, le volume intérieur du récipient augmente. A volume de moule constant, si le volume de verre augmente, la capacité du récipient diminue. De même, des variations de forme (hauteur, ovalisation du corps etc.) peuvent avoir une influence sur la capacité des récipients. Pour mesurer les caractéristiques volumiques des moules, le brevet FR 2 717 574 enseigne un procédé et un dispositif de jaugeage du volume intérieur d'un moule de verrerie.

Pour mesurer la capacité des récipients, il est connu par exemple une machine commercialisée par la société AGR international, Inc. (http://www.agrintl.com/products/view/10/Fill-Height-Tester), reposant sur le principe de la pesée. Cette machine comporte un marbre de pesée sur lequel est en appui le récipient vide reposant par son fond, en équilibre statique par gravité sur un plan de pose horizontal. Ce récipient est ensuite rempli d'un liquide de masse volumique connue jusqu'à un niveau nominal considéré par rapport au plan d'appui délimité par le marbre de pesée. Le remplissage du récipient au niveau nominal est réalisé en remplissant le récipient au-dessus du niveau nominal et en retirant le volume excessif par une pipette prenant appui sur la surface de la bague du récipient de sorte que l'orifice de la pipette se trouve situé au niveau nominal par rapport au plan d'appui. Cette machine réalise par pesée, à une température connue, la mesure de la quantité de liquide réellement contenue à l'intérieur du récipient et correspondant à la capacité effective du récipient.

Un inconvénient de cette machine concerne la durée pour réaliser cette mesure. De plus, cette machine présente l'inconvénient de ne pas pouvoir réaliser d'autres mesures dimensionnelles si ce n'est le poids à vide du récipient. Cette machine vient ainsi en complément des appareils automatiques de contrôle dimensionnel, du type optique ou par palpeurs mécaniques, qui ne permettent pas de mesurer la capacité des récipients.

Il est également connu par le document US 2014/211980 une méthode et un appareil à rayons X pour mesurer le volume d'un liquide remplissant partiellement une bouteille en détectant notamment la surface du liquide à l'intérieur de la bouteille. Si cette méthode permet de mesurer le volume d'un liquide à l'intérieur d'une bouteille, cette technique ne permet pas de mesurer la capacité réelle d'une bouteille d'une part, et selon les conditions normalisées de mesure d'autre part.

La demande de brevet US 2010/303287 décrit un appareil à rayons X adapté pour déterminer si un objet contient un liquide. Si un tel document permet de mesurer le volume de liquide contenu à l'intérieur d'une bouteille, la technique décrite par ce document présente les mêmes inconvénients que la demande de brevet US 2014/211980. Dans le même sens, la demande de brevet WO 2013/185816 décrit une méthode et un système à rayons X pour détecter des défauts dans des récipients ou dans leur contenu. Cette méthode ne permet pas de mesurer la capacité réelle d'une bouteille selon les conditions normalisées de mesure. En outre, ces techniques ne permettent pas de guider les opérateurs dans le pilotage du procédé de formage mis en oeuvre par l'installation de formage.

Un autre critère de qualité des récipients en verre concerne le rendu de reliefs aménagés sur les récipients soit pour des fonctions esthétiques tels que les blasons ou les gravures décoratives soit pour des fonctions techniques (texte, code ou autre inscription de contenance, de numéro de moule, de numéro de lot, de marque, de modèle) ou soit pour des fonctions mécaniques tel que la contre-bague ou filet de bouchon, ergot ou encoche de positionnement, stries de contact fond, garde étiquette.

Le rendu des reliefs est le fait que le relief relativement à une surface lisse moyenne ou d'arrière-plan est suffisant :
- soit pour une lecture humaine (aspect esthétique ou lecture d'information importante) ;
- soit pour une lecture automatique (cas des numéros de moules au jable codés sous forme de points ou perles) ;
- soit pour une utilisation mécanique comme un cran de calage pour l'orientation du récipient.

Le rendu des reliefs dépend de plusieurs paramètres :
- l'usure du moule c'est à dire donc la baisse du niveau positif ou négatif d'une empreinte du moule ;
- l'encrassement d'une empreinte du moule, par de la crasse obstruant l'empreinte du moule empêchant l'entrée du verre dans l'empreinte ;
- la thermique du verre, qui s'il est trop froid à l'endroit de l'empreinte, est trop visqueux pour pénétrer dans l'empreinte ;
- l'obstruction des évents servant à laisser échapper l'air pris entre l'épreinte et le verre, ou bien l'insuffisance de vide lorsque les évents nécessitent leur raccordement au vide.

L'estimation du rendu des reliefs qui est toujours très partielle, est souvent visuelle et subjective. Au plus, les rares mesures sont faites manuellement ou au microscope optique, ou avec des palpeurs par les services qualité. Ces mesures sont trop tardives pour servir au pilotage du procédé de formage. La plupart du temps, il n'existe pas de principe de mesure du rendu de relief standardisé.

Un autre critère de qualité des récipients en verre concerne la géométrie interne de goulot. En effet, particulièrement selon le procédé soufflé-soufflé, la surface interne du goulot n'est pas formée par un moule mais par l'air en pression.

Les contraintes techniques sur le goulot sont fortes en raison de l'emploi futur des récipients. Ainsi, la possibilité d'introduire une canule de remplissage sera garantie si un diamètre minimum est respecté sur la hauteur du goulot. En réalité il faut que le goulot puisse contenir un cylindre solide droit de diamètre suffisant. Cette vérification s'appelle le « brochage ».

Le diamètre au niveau de la surface de bague ou juste en-dessous est appelé le « diamètre à l'ouverture ». On mesure couramment le diamètre intérieur d'un cylindre sur une profondeur donnée sous la surface de bague, par exemple 5 mm, dont le diamètre doit être compris dans un intervalle de tolérance sur ladite profondeur. Ceci est nécessaire lorsque le récipient est prévu pour être fermé par un bouchon formant l'étanchéité par son contact sur la surface interne du haut du goulot.

Lorsque le récipient est destiné à être fermé par un bouchon élastique par exemple en liège, alors sur toute la hauteur du bouchon en place, par exemple sur 50 mm, les diamètres doivent avoir un profil donné appelé « profil interne » ou « profil de débouchage » qui est une fonction liant le diamètre interne avec la profondeur.

L'état de la technique a proposé diverses solutions techniques pour réaliser de telles inspections. Par exemple, le brevet GB 1 432 120 décrit un dispositif pour inspecter les récipients comportant plusieurs postes de contrôle dont l'un vise à contrôler la conformité dimensionnelle des bagues et des cols des récipients. Ce poste de contrôle comporte un équipage mobile entraîné par un système de motorisation selon un mouvement alternatif par rapport au bâti du dispositif, dans une direction de déplacement parallèle à l'axe de symétrie des récipients. Cet équipage mobile est équipé d'un calibre externe de contrôle de l'extérieur de la bague des récipients et d'un calibre interne de contrôle de l'intérieur de la bague et du col des récipients.

Un inconvénient d'un tel dispositif connu est le risque de choc violent entre la tête d'inspection et le récipient, risquant de détériorer le récipient ou le calibre. Un autre inconvénient de ce type de contrôle est qu'il ne mesure par les diamètres, mais vérifie seulement l'entrée d'un cylindre. Il ne permet donc pas la mesure du profil interne.

Pour les dispositifs actuels de mesure du profil interne sur des récipients échantillon, il est nécessaire d'introduire dans le goulot, des palpeurs articulés, au nombre de deux en position opposée, plus rarement trois à 120°. Les deux palpeurs constituent deux branches articulés en pince. Les extrémités basses de la pince sont mises en contact de la surface interne par un ressort. Leur écartement donne le diamètre interne. On fait alors tourner l'un par rapport à l'autre la pince et le récipient pour avoir plusieurs diamètres sur 360°, et l'on peut recommencer la mesure à d'autres profondeurs. L'inconvénient de ces palpeurs est leur lenteur, leur fragilité, leur usure, leur manque de précision, car en particulier il n'est jamais garanti qu'ils mesurent des diamètres et non pas des cordes d'arcs. De plus, tout contact avec des récipients chauds est à éviter.

Il existe bien d'autres critères de qualité des récipients en verre comme ceux relatifs aux dimensions fonctionnelles de la bague des récipients, à la planéité de la surface de la bague des récipients, à la verticalité des récipients, totale ou prise au niveau du col ou du corps des récipients , etc.

Les diamètres extérieurs et l'ovalisation de la paroi, la hauteur du récipient, la verticalité du corps, du col ou globale du récipient, la planéité des bagues, les diamètres internes des goulots sont mesurés à l'aide d'appareils « multi contrôles ». Il est à noter que ces appareils de mesure des récipients par prélèvement utilisent essentiellement soit des palpeurs mécaniques soit des détections optiques. Contrairement à ce que pourrait penser l'homme du métier, le fait que les récipients sont en verre transparent ne permet pas de mesurer aisément la surface interne par des procédés optiques. C'est la raison pour laquelle, les mesures des diamètres internes des goulots sont réalisées avec des dispositifs de palpeurs mécaniques même lorsque les autres mesures sont optiques.

L'analyse de l'état de la technique conduit à constater que le contrôle de la qualité des récipients en verre nécessite la mise en oeuvre de multiples dispositifs de contrôle ou de mesure. De plus, ces dispositifs de contrôle ou de mesure ne permettent pas d'obtenir des mesures précises, répétitives et rapides. Enfin, ces dispositifs de contrôle ou de mesure ne sont pas en mesure de donner une information suffisamment complète pour déterminer les corrections à apporter aux paramètres de contrôle de l'installation de formage des récipients en verre.

La présente invention vise à remédier aux inconvénients de l'art antérieur en proposant une méthode de contrôle de la qualité des récipients en verre, conçue pour réaliser à l'aide d'une seule machine, des mesures précises, répétitives et rapides et adaptée pour donner une information plus complète sur les corrections à apporter aux paramètres de contrôle d'un procédé de formage de récipients en verre d'une installation de formage.

Un autre objet de l'invention est de proposer une nouvelle méthode permettant de contrôler en tant que la qualité des récipients en verre, aussi bien la répartition du verre de ces récipients, que la capacité de ces récipients ou le rendu de reliefs présentés par de tels récipients en verre.

Un autre objet de l'invention est de proposer une nouvelle méthode donnant la possibilité de contrôler toujours par une seule machine, de nombreux autres critères de qualité des récipients en verre.

Un autre objet de l'invention est de proposer une méthode de contrôle de la qualité des récipients, adaptée pour être mise en oeuvre à n'importe quel moment du procédé de formage des récipients mais avantageusement plus tôt après leur formage, les récipients présentant d'ailleurs encore une haute température.

Pour atteindre de tels objectifs, la méthode vise à contrôler un procédé de formage de récipients en verre mettant en oeuvre une installation avec plusieurs sections de formage distinctes dans chacune desquelles au moins une paraison de verre fondu est en premier lieu mise en forme d'une ébauche dans au moins un moule ébaucheur, puis en second lieu mise en forme finale dans au moins un moule finisseur.

Selon l'invention, la méthode comporte les étapes suivantes :
- prélever un récipient dit échantillon issu d'un moule ébaucheur identifié et d'un moule finisseur identifié ;
- déposer le récipient échantillon sur un porte-échantillon d'un appareil de tomographie par rayons X assisté par ordinateur ;
- acquérir au moyen de l'appareil de tomographie, plusieurs images radiographiques du récipient échantillon sous des angles de projection différents ;
- transmettre les images radiographiques à un calculateur ;
- fournir au calculateur, la position du récipient échantillon dans le moule finisseur, dans un repère moule ;
   - analyser les images radiographiques par le calculateur pour :
      ▪ construire dans un repère virtuel, un modèle numérique tridimensionnel du récipient échantillon à partir des images radiographiques ;
      ▪ déterminer la position du modèle numérique tridimensionnel par rapport à la position du récipient échantillon dans le repère moule ;
- et analyser le modèle numérique tridimensionnel pour déterminer au moins un indicateur de qualité du récipient échantillon en relation d'au moins une région du récipient échantillon, permettant d'en déduire une information d'ajustement pour au moins un paramètre de contrôle du procédé de formage en relation du moule du récipient échantillon.

De plus, la méthode selon l'invention peut comporter en outre en combinaison au moins l'une et/ou l'autre des caractéristiques additionnelles suivantes :
- pour déterminer la position du modèle numérique tridimensionnel par rapport à la position du récipient échantillon dans le repère moule, une méthode mise en oeuvre par un opérateur consiste à repérer un relief de repérage sur le récipient échantillon et à déposer le récipient échantillon sur le porte-échantillon de manière que son relief de repérage soit positionné par rapport à un dispositif de repérage visuel ou mécanique du porte-échantillon ;
- pour déterminer la position du modèle numérique tridimensionnel par rapport à la position du récipient échantillon dans le repère moule, une autre méthode consiste à :
   ▪ choisir un relief de repérage sur le récipient échantillon dont la position est connue dans le repère moule ;
   ▪ localiser sur le modèle numérique tridimensionnel, le relief de repérage virtuel correspondant au relief de repérage choisi ;
   ▪ et déterminer la position du relief de repérage virtuel dans le repère virtuel pour en déduire la position du modèle numérique tridimensionnel dans le repère moule ;
- selon une variante avantageuse pour construire le modèle numérique tridimensionnel, la méthode consiste à prendre en compte le porte-échantillon de manière à disposer d'un axe vertical virtuel s'étendant perpendiculairement par rapport au plan de pose virtuel du récipient échantillon sur le porte-échantillon et à assurer une rotation relative autour de l'axe vertical virtuel, du modèle numérique tridimensionnel afin d'amener le relief de repérage virtuel dans une position correspondant à la position du relief de repérage dans le repère moule ;
- il est avantageux d'identifier le moule ébaucheur et/ou le moule finisseur d'où provient le récipient échantillon prélevé par un numéro de moule ou d'emplacement et de mettre à disposition ce numéro de moule ou ce numéro d'emplacement en relation de l'indicateur de qualité du récipient échantillon ;
- pour identifier le moule ébaucheur et/ou le moule finisseur d'où est issu le récipient échantillon portant un relief qui indique le numéro de moule ou d'emplacement sous forme d'un code ou alphanumérique, la méthode consiste à :
   ▪ assurer la lecture du relief porté par le récipient échantillon et à communiquer le numéro lu au calculateur ;
   ▪ ou à analyser le modèle numérique tridimensionnel du récipient échantillon, en recherchant l'endroit d'un relief virtuel correspondant au relief du récipient échantillon, et à lire ce relief virtuel pour sa mise à disposition au calculateur ;
- selon une application préférée, le récipient échantillon est prélevé au plus tard avant l'entrée de l'arche de recuisson de l'installation ;
- avantageusement, le procédé consiste à déterminer un indicateur de qualité du récipient échantillon, permettant de déduire une information d'ajustement pour au moins un paramètre de contrôle du procédé de formage des récipients pour les moules identifiés, parmi :
   ▪ le poids ou la forme de la paraison de verre chargée dans le moule ébaucheur identifié ;
   ▪ la position ou la vitesse de la paraison de verre à son chargement dans le moule ébaucheur identifié ;
   ▪ une synchronisation ou vitesse ou force dans le mouvement des mécanismes des poinçons de perçage, des moules identifiés, des transferts de l'ébauche, des pinces d'extraction ;
   ▪ le refroidissement des moules identifiés ou d'un poinçon associé ;
   ▪ une pression de soufflage ou de pressage pour les moules identifiés ;
   ▪ le remplacement d'un moule identifié ;
- selon un exemple d'application, la méthode consiste à déterminer en tant qu'indicateur de qualité du récipient échantillon, la répartition du verre du récipient échantillon ;
- selon un autre exemple d'application, la méthode consiste à déterminer en tant qu'indicateur de qualité du récipient échantillon, au moins une mesure de volume du récipient échantillon prise parmi, la capacité du récipient échantillon, le volume de l'enveloppe du récipient échantillon et le volume de verre du récipient échantillon ;
- selon un autre exemple d'application, la méthode consiste à déterminer en tant qu'indicateur de qualité du récipient échantillon, le rendu de reliefs aménagés sur le récipient échantillon ;
- selon un autre exemple d'application, la méthode consiste à déterminer en tant qu'indicateur de qualité du récipient échantillon, la géométrie interne de goulot du récipient échantillon ;
- selon un autre exemple d'application, la méthode consiste à déterminer en tant qu'indicateur de qualité du récipient échantillon, la planéité de la surface de bague du récipient échantillon ;
- selon un autre exemple d'application, la méthode consiste à déterminer en tant qu'indicateur de qualité du récipient échantillon, des diamètres extérieurs du corps du récipient échantillon ;
- pour la détermination de la répartition de verre en tant qu'indicateur de qualité du récipient échantillon, la méthode consiste à déterminer la position du centre de masse du modèle numérique tridimensionnel ou d'une portion dudit modèle, et en comparant cette position à une position de référence ;
- pour la détermination de la répartition de verre en tant qu'indicateur de qualité du récipient échantillon, la méthode consiste à déterminer l'épaisseur de la paroi de verre sur au moins une région du récipient échantillon, en recherchant dans cette région la position d'une zone avec une épaisseur supérieure à une valeur prédéfinie et/ou une épaisseur inférieure à une valeur prédéfinie, éventuellement en déterminant l'étendue de ladite zone, et/ou en recherchant la présence et la position de l'endroit de la paroi présentant le minimum ou le maximum d'épaisseur dans ladite région ;
- pour la détermination de la répartition de verre en tant qu'indicateur de qualité du récipient échantillon la méthode consiste :
   ▪ à déterminer le volume de verre contenu dans au moins deux régions du modèle numérique tridimensionnel divisé soit par un plan de section verticale contenant l'axe vertical virtuel du modèle numérique tridimensionnel ou soit par un plan de section horizontale perpendiculaire audit axe vertical virtuel ;
   ▪ et à comparer lesdits volumes à des valeurs de volume de référence et/ou entre plusieurs régions d'un même récipient échantillon, et/ou entre plusieurs récipients échantillons ;
- pour la détermination du rendu de reliefs aménagés sur le récipient échantillon en tant qu'indicateur de qualité du récipient échantillon, la méthode consiste à :
   ▪ positionner au moins un plan de coupe sur le modèle numérique tridimensionnel du récipient échantillon de manière qu'il sectionne au moins une partie d'un relief virtuel de la surface externe dudit modèle ;
   ▪ déterminer dans le plan de coupe, la courbe représentative de la section du relief virtuel ;
   ▪ superposer au moins partiellement à la courbe représentative, une courbe d'altitude zéro représentant la courbe de la surface externe du récipient échantillon dépourvu dudit relief virtuel ;
   ▪ comparer la courbe représentative à la courbe d'altitude zéro, en calculant comme critère de rendu du relief virtuel au moins une des grandeurs suivantes :
      - une distance entre la courbe représentative et la courbe d'altitude zéro ;
      - un écart de pente à une position donnée entre la courbe représentative et la courbe d'altitude zéro ;
      - une variation de la pente de la courbe représentative ;
      - une aire délimitée par la courbe représentative et la courbe d'altitude zéro ;
- pour la détermination du rendu de reliefs aménagés sur le récipient échantillon en tant qu'indicateur de qualité du récipient échantillon, une variante consiste à :
   ▪ déterminer la surface représentative du relief comme une portion de surface externe du modèle numérique tridimensionnel dans la zone d'intérêt contenant au moins une partie d'un relief virtuel ;
   ▪ superposer au moins partiellement à la surface externe de la zone d'intérêt, une surface d'altitude zéro représentant la surface de la zone d'intérêt dépourvu dudit relief virtuel ;
   ▪ comparer la surface représentative à la surface d'altitude zéro, en calculant comme critère de rendu du relief au moins une des grandeurs suivantes :
      - une distance entre la surface d'altitude zéro et la surface représentative ;
      - l'écart de pente à une position donnée entre la surface d'altitude zéro et la surface représentative ;
      - une variation des pentes de la surface représentative ;
      - des volumes délimités par la surface d'altitude zéro et la surface représentative ;
- pour la détermination du rendu de reliefs aménagés sur le récipient échantillon en tant qu'indicateur de qualité du récipient échantillon, une autre variante consiste à :
   ▪ déterminer la surface représentative d'un relief virtuel comme une portion de surface externe du modèle numérique tridimensionnel dans la zone d'intérêt contenant au moins une partie du relief virtuel correspondant au relief du récipient échantillon ;
   ▪ superposer au moins partiellement à la surface externe de la zone d'intérêt, une surface de relief théorique représentant la surface de la zone d'intérêt si le relief virtuel est rendu correctement ;
   ▪ comparer la surface représentative à la surface théorique, en calculant comme critère de rendu du relief, au moins une des grandeurs suivantes :
      - une distance entre la surface représentative et la surface théorique ;
      - un écart de pente à une position donnée entre les surfaces ;
      - des volumes délimités par les surfaces ;
- pour la détermination du rendu de reliefs aménagés sur le récipient échantillon en tant qu'indicateur de qualité du récipient échantillon une autre variante consiste à :
   ▪ sélectionner sur le modèle numérique tridimensionnel, un relief virtuel correspondant à un relief à fonction technique dont la position est connue ;
   ▪ positionner un plan de section de manière qu'il coupe ledit relief dans un plan de section correspondant à un plan de conception ;
   ▪ obtenir une courbe représentative de la section du relief virtuel ;
   ▪ mesurer sur cette courbe représentative, un rayon de courbure et/ou un angle, une longueur, une distance à une courbe d'altitude zéro ;
   ▪ comparer la mesure à des valeurs de tolérance prédéfinies.
- pour la détermination de la capacité du récipient échantillon en tant qu'indicateur de qualité du récipient échantillon, la méthode consiste à :
   ▪ déterminer la surface interne du modèle numérique tridimensionnel du récipient échantillon ;
   ▪ déterminer un plan de niveau de remplissage sur le modèle numérique tridimensionnel du récipient échantillon, le niveau de remplissage étant soit le plan de surface de bague virtuel soit un plan de niveau de remplissage nominale ;
   ▪ et mesurer par calcul, le volume interne du modèle numérique tridimensionnel du récipient échantillon délimité par la surface interne et le plan de niveau de remplissage, cette mesure étant la capacité du récipient échantillon ;
- pour la détermination du volume de l'enveloppe du récipient échantillon en tant qu'indicateur de qualité du récipient échantillon, la méthode consiste à :
   ▪ déterminer la surface externe du modèle numérique tridimensionnel du récipient échantillon ;
   ▪ déterminer un plan fermeture de volume comme étant le plan de surface de bague ou le plan inférieur de joint de moule de bague ;
   ▪ et mesurer par calcul le volume intérieur délimité par la surface externe et le plan de fermeture comme étant le volume de l'enveloppe du récipient échantillon ;
- pour la détermination du volume de verre du récipient échantillon en tant qu'indicateur de qualité du récipient échantillon, la méthode consiste à déterminer le volume de la paroi du modèle numérique tridimensionnel du récipient échantillon ;
- une variante avantageuse consiste à analyser le modèle numérique tridimensionnel en recherchant des bulles correspondant à des manques de matière entre la surface interne et la surface externe, et à mesurer les volumes desdites bulles, qui sont ensuite soustraits au volume de la paroi du modèle numérique tridimensionnel, déterminé entre la surface interne et la surface externe, en vue d'obtenir un volume correspondant au volume de verre de la paraison chargée dans le moule ébaucheur identifié dont l'ébauche a été transférée dans le moule finisseur dont est issue le récipient échantillon ;
- selon une application avantageuse, la méthode consiste à :
   ▪ considérer comme étant une mesure du volume de la paraison chargé dans le moule ébaucheur, le volume de verre du modèle numérique tridimensionnel, avec la prise en compte ou non des manques de matière ;
   ▪ considérer le volume intérieur délimité par la surface externe du modèle numérique tridimensionnel et un plan de fermeture comme étant une mesure du volume interne du moule finisseur ;
   ▪ considérer le volume délimité par la surface interne du modèle numérique tridimensionnel et un plan de niveau de remplissage comme étant une mesure de la capacité du récipient échantillon ;
   ▪ déduire des mesures de la capacité du récipient échantillon et du volume interne du moule finisseur, le volume de la paraison à charger dans le moule ébaucheur dont est issu le récipient échantillon ;
   ▪ et décider lorsque la capacité du récipient échantillon n'est pas conforme, de modifier le poids de la paraison pour au moins le moule ébaucheur dont est issu le récipient échantillon ou de remplacer le moule finisseur ;
- pour la détermination de la géométrie du goulot du récipient échantillon en tant qu'indicateur de qualité du récipient échantillon, la méthode consiste à :
   ▪ déterminer sur le modèle numérique tridimensionnel la surface interne correspondant au moins à celle du goulot ;
   ▪ positionner au moins un plan de coupe parallèle à un plan de pose virtuel ;
   ▪ mesurer dans ce plan plusieurs diamètres de la surface interne et déterminer le minimum et/ou le maximum dans le plan de coupe ;
- avantageusement, la méthode vise à déterminer comme indicateur de la géométrie du goulot :
   ▪ le diamètre à l'ouverture ;
   ▪ et/ou le diamètre de brochage ;
   ▪ et/ou le profil interne du récipient échantillon ;
- pour la détermination de la planéité de la surface de bague du récipient échantillon en tant qu'indicateur de qualité du récipient échantillon, la méthode consiste à :
   ▪ déterminer à partir du modèle numérique tridimensionnel, une courbe tridimensionnelle fermée ou une surface annulaire représentative de la surface de bague ;
   ▪ positionner un plan de référence de surface de bague en relation de la courbe tridimensionnelle fermée ou de la surface annulaire ;
   ▪ et mesurer les écarts entre le plan de référence et la courbe tridimensionnelle fermée ou la surface annulaire ;
- pour la détermination des diamètres extérieurs du corps du récipient échantillon en tant qu'indicateur de qualité du récipient échantillon, la méthode consiste à :
   ▪ déterminer à partir du modèle numérique tridimensionnel, la surface externe correspondant au moins la partie du récipient échantillon pour laquelle un diamètre extérieur est à mesurer ;
   ▪ positionner un plan de coupe parallèle au plan de pose virtuel du modèle selon au moins une hauteur du récipient ;
   ▪ mesurer plusieurs diamètres dans ce plan de coupe par rapport à la surface externe et à comparer ces mesures à des valeurs de référence.

Ainsi, la méthode selon l'invention permet de réaliser, en plus de mesures impossibles auparavant comme la répartition du verre, ou avec des appareils séparés (contrôle de capacité et jaugeage des moules), toutes les mesures effectuées par les machines de métrologie verrière par palpeurs et/ou capteurs optiques de l'art antérieur.

L'invention concerne également une machine pour contrôler un procédé de formage de récipients en verre mettant en oeuvre une installation avec plusieurs sections de formage distinctes dans chacune desquelles au moins une paraison de verre fondu est en premier lieu mise en forme d'une ébauche dans au moins un moule ébaucheur, puis en second lieu mise en forme finale dans au moins un moule finisseur.

Selon l'invention, la machine comporte :
- un appareil de tomographie par rayons X assisté par ordinateur, apte à réaliser plusieurs images radiographiques sous des angles de projection différents d'un récipient échantillon placé sur un porte-échantillon dudit appareil ;
- un dispositif pour connaître la position du récipient échantillon dans le moule finisseur, selon un repère du moule ;
- un calculateur relié au dispositif et à l'appareil de tomographie et configurer pour analyser les images radiographiques pour :
   ▪ construire dans un repère virtuel, un modèle numérique tridimensionnel du récipient échantillon à partir des images radiographiques ;
   ▪ déterminer la position du modèle numérique tridimensionnel par rapport à la position du récipient échantillon dans le repère machine ;
   ▪ analyser le modèle numérique tridimensionnel pour déterminer au moins un indicateur de qualité du récipient échantillon en relation d'au moins une région du récipient échantillon, permettant d'en déduire une information d'ajustement pour au moins un paramètre de contrôle du procédé de formage en relation du moule du récipient échantillon ;
- et un système pour délivrer au moins l'indicateur de qualité du récipient échantillon en relation d'au moins une région du récipient échantillon.

De plus, la machine selon l'invention peut comporter en outre en combinaison au moins l'une et/ou l'autre des caractéristiques additionnelles suivantes :
- le système pour délivrer au moins l'indicateur de qualité du récipient échantillon en relation d'au moins une région du récipient échantillon comporte un système d'affichage pour l'indicateur de qualité en relation de l'identité du moule finisseur ;
- le système pour délivrer au moins l'indicateur de qualité du récipient échantillon en relation d'au moins une région du récipient échantillon comporte une connexion pour transmettre à un système de commande de l'installation de formage, l'indicateur de qualité en relation de l'identité du moule finisseur ;
- un système fournissant au calculateur, le numéro de moule ou d'emplacement du récipient échantillon.

L'invention concerne également une installation de formage de récipients en verre comportant plusieurs sections de formage distinctes dans chacune desquelles au moins une paraison de verre fondu est en premier lieu mise en forme d'une ébauche dans au moins un moule ébaucheur, puis en second lieu mise en forme finale dans au moins un moule finisseur.

Selon l'invention, l'installation comporte une machine conforme à l'invention disposée à la sortie des moules finisseurs.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue schématique de dessus montrant une machine de contrôle conforme à l'invention positionnée à titre d'exemple en sortie d'une installation de formage de récipients.
La **Figure 2** illustre de manière schématique, en vue de côté selon un axe transversal **X,** une installation de moulage connue en soi.
La **Figure 3** est une vue schématique de dessus montrant des moules finisseurs en cours d'ouverture venant de former des récipients en verre.
La **Figure 4** est une vue schématique en perspective d'un exemple d'un modèle numérique tridimensionnel d'un récipient obtenu à l'aide d'un appareil de tomographie assistée par ordinateur.
La **Figure 4A** est une vue en plan montrant un exemple d'une image d'un code issu d'une modélisation d'un récipient.
La **Figure 5** est une vue schématique en coupe-élévation d'un exemple d'un modèle numérique tridimensionnel d'un récipient.
Les **Figures 6** et **7** sont des vues de dessus du modèle numérique tridimensionnel dans deux positions caractéristiques par rapport à un repère virtuel.
Les **Figures 8** et **9** sont des vues en coupe-élévation du modèle numérique tridimensionnel montrant la position du centre de masse par rapport à un centre de masse.
La **Figure 10** est une vue schématique en coupe-élévation passant par l'axe vertical virtuel **Zv** du modèle numérique tridimensionnel donnant la répartition des volumes de verre **Vr** selon l'axe vertical virtuel **Zv.**
La **Figure 11** est une vue schématique en plan passant par l'axe vertical virtuel **Zv** du modèle numérique tridimensionnel donnant la répartition des volumes de verre répartis selon huit zones A à H.
La **Figure 12** est une vue schématique d'un récipient explicitant la définition de la capacité d'un récipient.
La **Figure 13A** est une vue d'un modèle numérique tridimensionnelle d'un récipient.
Les **Figures 13B** à **13D** sont des vues en coupe selon les lignes C-C de la **Fig. 13A** et illustrant différentes méthodes d'inspection du rendu pour des reliefs.
La **Figure 14** est une vue en coupe d'un modèle numérique tridimensionnelle montrant le goulot d'un récipient et illustrant la méthode de contrôle de la géométrie interne du goulot d'un récipient.
La **Figure 14A** est une courbe représentant les valeurs du diamètre intérieur Di du goulot suivant l'axe vertical Zv du repère virtuel.
La **Figure 15** est une vue en coupe d'un modèle numérique tridimensionnelle montrant le goulot d'un récipient et illustrant la méthode de contrôle de la planéité de la surface de la bague d'un récipient.
La **Figure 16** est une vue en perspective d'un modèle numérique tridimensionnelle d'un récipient et illustrant la méthode de contrôle des diamètres extérieurs du corps d'un récipient.

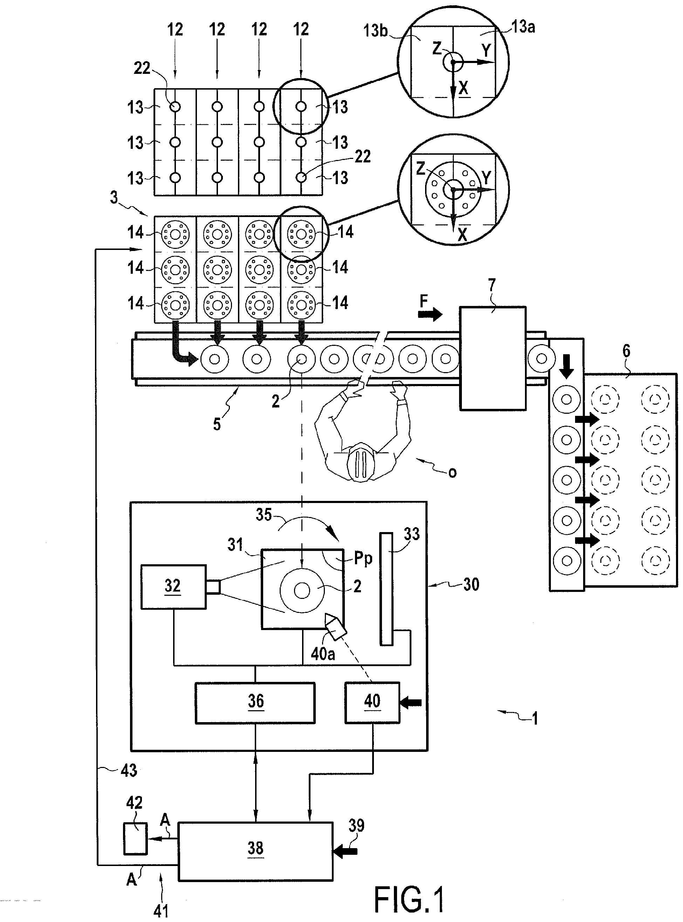

Tel que cela ressort plus précisément de la **Fig. 1****,** l'objet de l'invention concerne une machine **1** permettant de contrôler un procédé de formage de récipients en verre **2** transparents ou translucides fabriqués par une installation de fabrication ou de formage **3** de tous types connus en soi. En sortie de l'installation de formage **3,** les récipients **2** tels que par exemple des bouteilles ou des flacons en verre, présentent une haute température typiquement comprise entre 300°C et 600°C.

De manière connue, les récipients **2** qui viennent d'être formés par l'installation **3** sont posés successivement sur un convoyeur de sortie **5** pour former une file de récipients. Les récipients **2** sont transportés en file par le convoyeur **5** selon une direction de transfert **F** afin de les acheminer successivement à différents postes de traitement et en particulier une arche de recuisson **6,** en amont de laquelle est placée une hotte de traitement de surface **7** constituant généralement le premier des postes de traitement après le formage.

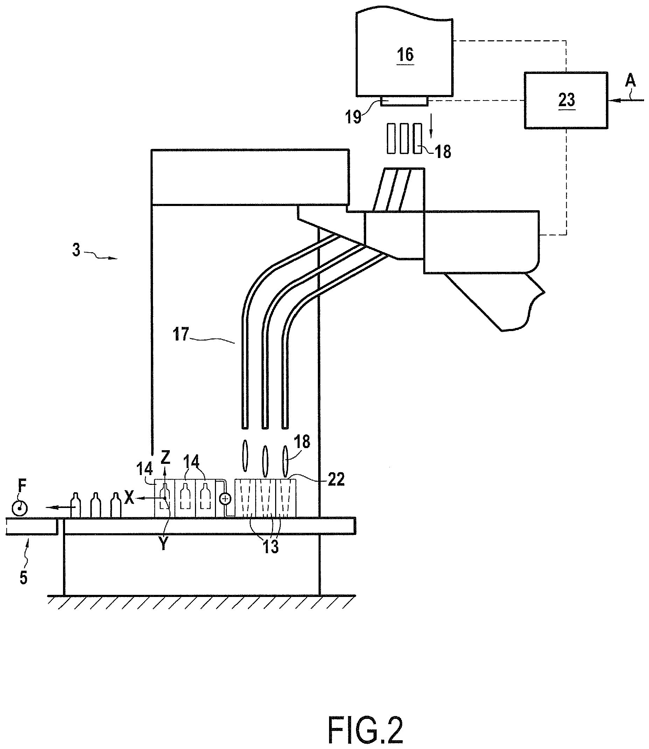

Les **Fig. 1** à **3** illustrent un exemple de réalisation d'une installation de formage **3** connue en soi et qui sera décrite de manière succincte pour uniquement permettre une compréhension du fonctionnement de la machine **1** conforme à l'invention en relation de l'installation de formage **3.**

L'installation **3** comporte plusieurs sections de formage distinctes **12** comportant chacune au moins un moule ébaucheur **13** et au moins un moule finisseur **14.** L'installation **3** comporte une source **16** de verre malléable, donc de verre chaud, et un distributeur **17** de paraisons de verre qui distribue, par gravité, des paraisons de verre malléable **18** à chaque moule ébaucheur **13.** De manière connue, la source **16** de verre malléable est un réservoir alimenté en verre fondu, au fond duquel se trouve une cuvette comportant une à quatre ouvertures circulaires. Un tube rotatif dont la hauteur est régulée, contrôle le débit de verre au-dessus de la cuvette, et un système de un à quatre plongeur(s) animé(s) d'un mouvement de va et vient, extrude le verre à travers les une à quatre ouvertures de la cuvette afin de délivrer par gravité, le verre malléable sous une forme de un à quatre chapelets en parallèles. Les chapelets de verre malléable sont définitivement séparés en gouttes indépendantes par un système de ciseaux **19** agencé à la sortie de la source de verre chaud **16** et qui est actionné à intervalles réguliers pour découper en tronçons le verre malléable issu de la source **16.**

Pour les installations comportant plusieurs (jusqu'à quatre) cavités de moulage par section, éventuellement plusieurs tronçons sont délivrés en parallèle simultanément. Dans la présente description, on appelle paraison **18** une goutte ou tronçon extrudé de verre malléable tel que débité par le système à ciseaux **19.** En langue anglaise, la paraison est, à ce stade d'un procédé de formage, appelée « gob ». Le verre malléable, au niveau de la découpe par le système à ciseaux **19,** présente généralement une température supérieure à 900°C, par exemple comprise entre 1 100 et 1 300°C. Cette paraison est globalement un cylindre plein de verre malléable ayant un volume et une longueur définis par le réglage de la source **16** coopérant avec la coupe du système à ciseaux **19.** En effet, le diamètre des paraisons est défini par celui des ouvertures de la cuvette. Le débit est contrôlé à la fois par la hauteur du tube qui agit sur le débit global et par les mouvements des un à quatre plongeurs, ce qui permet de varier le débit séparément pour chaque ouverture de la cuvette. L'intervalle de temps entre deux actionnements du système à ciseaux **19** détermine la longueur de la paraison. Pour résumer la longueur, le poids et le volume de chaque paraison sont déterminés par les paramètres de la source **16** (le tube et les plongeurs) et le système de ciseaux **19.** La source **16** de verre malléable est agencée au-dessus des moules ébaucheurs **13** pour permettre la distribution par gravité des paraisons qui sont chargées au travers d'ouvertures **22** aménagées dans les faces supérieures des moules ébaucheurs **13.**

Le distributeur **17** s'étend selon plusieurs ramifications entre la source **16** de verre chaud et les moules ébaucheurs **13** de chacune des sections de formage. Généralement, la source **16** de verre chaud, par l'intermédiaire du système de ciseaux **19,** délivre simultanément autant de paraisons qu'il y a de moules ébaucheurs (respectivement de moules finisseurs) dans une section de formage. On comprend donc que les sections de formage sont alimentées en paraisons successivement l'une après l'autre.

Le distributeur **17** recueille donc les paraisons découpées par le système de ciseaux **19** et les conduit vers chacun des moules ébaucheurs **13** de chacune des sections de formage **12** selon une trajectoire de chargement correspondante. Les trajectoires de chargement pour les différents moules ébaucheurs **13** comportent des portions communes et des portions spécifiques. Une portion spécifique est une portion de la trajectoire de chargement correspondant à un moule ébaucheur **13** qui est suivie uniquement par les paraisons qui sont dirigées par le distributeur vers ce moule ébaucheur.

Le distributeur **17** comporte donc des moyens d'aiguillage qui est un genre de goulotte ou groupe de goulottes pivotant, puis de guidage des paraisons comportant des goulottes et des déflecteurs en fin de course, au-dessus des moules ébaucheurs. Notamment la position des déflecteurs par rapport aux moules ébaucheurs associés détermine en partie la position et orientation du chargement de chaque paraison dans lesdits moules ébaucheurs. Dans le distributeur, les goulottes, les déflecteurs et aiguillages déterminent la trajectoire de chargement des paraisons.

Les installations de formage de récipients en verre mettent en oeuvre différents procédés combinant des étapes de remplissage, puis de pressage et/ou soufflage successifs. Pour la clarté de la description, l'exemple est pris du formage des récipients selon les procédés connus dits de pressé-soufflé ou de soufflé-soufflé.

Dans les installations de formage de récipients, chaque section de formage **12** peut comporter plusieurs moules, par exemple deux moules dont l'un est un moule ébaucheur **13** et l'autre est un moule finisseur **14.** Chaque section **12** peut comporter un ensemble de moules ébaucheurs et un ensemble de moules finisseurs associés. On comprend dans ce cas, qu'une paraison donnée est guidée par le distributeur **17** vers un moule ébaucheur, par exemple un moule ébaucheur **13** de la section de formage où la paraison subit une première opération de formage, dite de perçage, effectuée par soufflage d'air comprimé ou par pénétration d'un poinçon. Un système de transfert (non représenté) est capable ensuite de prélever la paraison ayant subi la première opération de formage, à savoir l'ébauche, dans le moule ébaucheur **13** pour l'emmener dans un moule finisseur **14** où l'ébauche peut subir au moins une deuxième opération de formage, la dernière opération dite de finition. Généralement, chaque moule ébaucheur ou finisseur d'une section de formage comporte deux demi-moules respectivement **13a, 13b** et **14a, 14b** qui sont mobiles l'un par rapport à l'autre selon une direction perpendiculaire à un plan de joint **P** par lequel les deux demi-moules **13a, 13b** et **14a, 14b** sont en contact dans une position fermée. Dans l'exemple illustré, le plan de joint **P** s'étend selon la direction verticale **Z** et la direction transversale **X.**

Une section **12** peut comporter un unique moule finisseur **14** recevant une ébauche d'un unique moule ébaucheur **13.** Cependant, comme évoqué plus haut, chacune des différentes sections de formage **12** peut comporter au moins deux moules finisseurs **14** distincts et autant de moules ébaucheurs **13.** On a illustré sur les **Figures** le cas de quatre sections de formage **12** décalées selon une direction longitudinale **Y** perpendiculaire à la direction transversale **X.** Selon cet exemple, chaque section de formage **12** comporte trois moules ébaucheurs **13** respectivement avant, central et arrière, (ou externe, central et interne) associés chacun à un moule finisseur **14** respectivement avant, central et arrière c'est-à-dire recevant chacun l'ébauche issue d'un moule ébaucheur **13.** Dans l'exemple illustré, les différents moules ébaucheurs **13** et respectivement les moules finisseurs **14** d'une même section sont décalées l'un par rapport à l'autre selon une direction transversale **X.** Dans l'exemple illustré, les moules finisseurs **14** d'une même section sont de forme identique, donc généralement destinées à former des récipients identiques, mais on pourrait prévoir des formes et poids différents.

Il est à noter que chaque moule finisseur **14** est identifié dans l'installation de formage par rapport aux autres moules finisseurs **14.** De même, chaque moule ébaucheur **13** est identifié dans l'installation de formage. Il est ainsi possible d'identifier la section de formage **12,** le moule ébaucheur **13** et le moule finisseur **14** d'où est issu chaque récipient **2.**

Dans une installation de formage de récipients en verre, chaque emplacement de moule ébaucheur **13** de chaque section porte selon différentes conventions possibles, un identifiant, par exemple un numéro ou une lettre. Par exemple, les trois emplacements pour la deuxième section de l'installation représentée à la **Fig. 1** peuvent être identifiés par les lettres **a, b** et **c** formant donc les emplacements **2a, 2b, 2c** pour désigner respectivement le moule avant, central et arrière de la section numéro 2, ces identifications seront appelés dans la suite de la description, « numéros d'emplacement ».

Par ailleurs, les moules finisseurs du fond ou du corps peuvent porter une empreinte afin d'imprimer en relief sur les récipients **2,** le numéro du moule par exemple entre 1 et 99 ou entre 1 et 128, etc. Une table de correspondance entre les numéros d'emplacement et les numéros de moule est en permanence disponible pour les opérateurs ou le système d'information de l'installation. Dans certaines installations, on emploie un marqueur laser comme décrit dans le brevet EP 2 114 840 B1 afin d'imprimer à chaque récipient encore chaud immédiatement après son formage, un code renseignant le numéro de moule ou le numéro d'emplacement.

Ainsi, les récipients portent généralement soit de manière codée (code barre, code à points, code Datamatrix) soit de manière alphanumérique, l'indication du numéro de moule ou du numéro d'emplacement. Pour relire ces numéros de moule ou d'emplacement portés par les récipients, il existe pour les lignes de fabrication, divers systèmes de lecture optique tels que décrits dans EP 1 010 126 ou EP 2 297 672 ou EP 2 992 315.

Ainsi, dans la présente description, on comprend qu'identifier le moule finisseur d'où provient un récipient échantillon revient donc à connaître ou bien le numéro d'emplacement ou bien le numéro de moule. Il est compris que l'identification du moule finisseur permet d'identifier directement le moule ébaucheur associé fournissant l'ébauche.

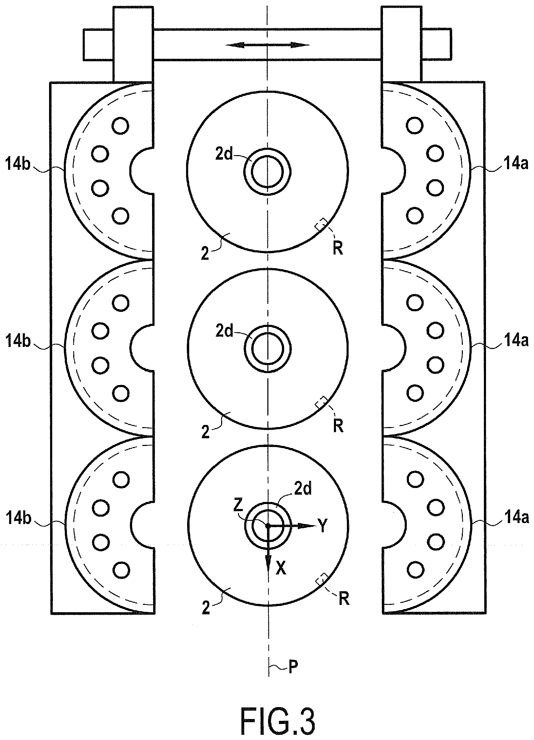

Il ressort également de la description qui précède que chaque moule ébaucheur **13** et chaque moule finisseur **14** présente un repère moule **X, Y, Z** permettant de localiser précisément chaque récipient dans ce repère moule (**Fig. 1** et **3**). En d'autres termes, chaque récipient **2** fabriqué peut ainsi être localisé dans ce repère moule **X, Y, Z** du moule ébaucheur **13** et du moule finisseur **14** d'où est issu chaque récipient **2.** L'axe vertical **Z** est l'axe de symétrie du récipient passant par l'axe de son goulot alors que l'axe transversal **X** est contenu dans le plan de joint du moule de sorte que le plan **XZ** est appelé le plan de joint **P** du moule. L'axe longitudinal **Y** positif est placé du côté de la demi coque droite du moule pour un observateur **O** situé devant l'installation de formage du côté des moules finisseurs **14** et du convoyeur de sortie **5.**

Dans les installations de formage, la commande et la synchronisation des opérations de formation des paraisons, coupe ciseau, mouvements des moules, mouvements des poinçons, soufflage, transferts, etc. sont effectuées au moyen d'un système de commande **23** au sens général, permettant de piloter les différents mécanismes nécessaires au fonctionnement de l'installation pour la mise en oeuvre du procédé de formage des récipients.

Conformément à une caractéristique de la méthode de contrôle conforme à l'invention, un récipient **2** dit échantillon issu d'un moule finisseur **14** est prélevé en sortie de ce moule finisseur **14.** Le récipient échantillon **2** est prélevé en tous endroits des différents postes de traitement après le formage. Selon une caractéristique avantageuse de mise en oeuvre, le récipient échantillon **2** est prélevé au plus tard avant l'entrée de l'arche de recuisson de l'installation. Dans ce cas, le récipient échantillon **2** présente une haute température typiquement comprise entre 300 et 600°C. Il est à noter que le moule finisseur **14** d'où est issu ce récipient échantillon est identifié comme expliqué précédemment c'est-à-dire que la section de formage **12** à laquelle appartient ce moule finisseur **14** est connu ainsi qu'est identifié le moule ébaucheur **13** qui a formé l'ébauche chargée ensuite dans ce moule finisseur.

Ce récipient échantillon **2** est destiné à être inspecté par la machine de contrôle **1** conforme à l'invention et plus précisément par un appareil de tomographie par rayons X assisté par ordinateur **30** faisant partie de cette machine. Typiquement, cet appareil de tomographie par rayons X assisté par ordinateur **30** comporte un porte-échantillon **31** sur lequel est déposé le récipient échantillon **2.**

Tel que cela ressort plus précisément de la **Fig. 1****,** un appareil de tomographie assistée par ordinateur **30** comporte de manière classique, dans une enceinte étanche aux rayons X, au moins une source **32** de génération de rayons X issus de son foyer d'émission et au moins un capteur **33** linéaire ou matriciel sensible aux rayons X. Le porte-échantillon **31** de l'appareil **30** sert de plan de pose mécanique **Pp** pour le récipient échantillon **2** et est adapté pour positionner entre la source **32** et le capteur **33,** le récipient échantillon **2** qui se trouve ainsi irradié par les rayons X. Par absorption et diffusion, la matière du récipient échantillon **2** atténue les rayons X qui la traversent en fonction de la masse atomique et de l'épaisseur de matière traversée. Le récipient échantillon **2** étant vide, seule la matière du récipient échantillon atténue les rayons X. Le capteur **33** sensible aux rayons X situé à l'opposé du tube par rapport au récipient échantillon **2,** reçoit les rayons X atténués, et délivre une image de l'atténuation que provoque la seule matière du récipient, c'est-à-dire une image radiographique **I** de la paroi du récipient échantillon **2.**

L'appareil **30** comporte également un système **35** pour créer un mouvement relatif entre le récipient échantillon **2** et le couple source **32** - capteur **33.** Classiquement, ce système **35** provoque un déplacement de valeur connue du récipient échantillon **2** par rapport au couple source **32** - capteur **33,** qui reste fixe. Avantageusement, ce système de déplacement **35** assure la rotation du récipient échantillon sur lui-même autour d'un axe de rotation qui de préférence mais non obligatoirement, est confondu avec l'axe vertical de symétrie du récipient échantillon.

L'appareil 30 comporte également une unité **36** de commande de la source **32,** du capteur **33** et du système de déplacement **35** permettant le fonctionnement de l'appareil et l'obtention des images radiographiques. Ainsi, cette unité de commande **36** assure un déplacement relatif connu du récipient échantillon **2** par rapport à la source **32** et au capteur **33** de manière à réaliser des projections du récipient échantillon suivant des angles variables. L'unité de commande **36** assure l'acquisition pendant ce déplacement, de plusieurs images radiographiques. Ainsi, le récipient échantillon **2** est déplacé entre chaque acquisition d'une image radiographique, de manière que chaque image radiographique soit une projection du récipient échantillon suivant des directions différentes entre elles. Les images radiographiques du récipient échantillon vide **2** acquises sont transmises à un calculateur **38** pour analyse et traitement.

Il est à noter que le capteur **33** peut présenter une hauteur de champ supérieure à la taille du récipient échantillon **2.** Le système de déplacement **35** est commandé pour assurer la rotation du récipient échantillon **2** sur lui-même typiquement sur un tour et l'unité **36** assure l'acquisition des différentes projections du récipient sur les 360° de rotation.

Selon une autre variante de réalisation, le capteur **12** peut présenter une hauteur de champ inférieure à la taille du récipient échantillon **2.** Selon cet exemple, le système de déplacement **35** est conçu pour assurer également un déplacement en translation verticale relatif entre le récipient échantillon **2** et la source **32** et/ou le capteur **33** pour analyser par balayage la totalité du récipient échantillon **2.**

Par exemple, le système de déplacement **35** assure la rotation du récipient échantillon **2** sur lui-même et une translation verticale du récipient échantillon **2** par rapport au couple source **32**-capteur **33,** qui reste fixe. Dans le cas où le capteur **33** est un capteur linéaire de champ horizontal, l'unité **36** pilote le système de déplacement pour positionner le récipient échantillon **2** de manière que l'extrémité haute du récipient échantillon soit positionnée dans le champ du capteur **33.** L'unité **36** pilote ensuite la rotation du récipient échantillon **2** sur un tour et assure l'acquisition des projections du récipient échantillon sur le capteur pendant ce tour. Le système de déplacement **35** déplace en translation vers le bas le récipient échantillon selon un pas incrémental avant la rotation du récipient échantillon sur lui-même et l'acquisition des projections du récipient échantillon. Les étapes de déplacement et d'acquisition sont renouvelées jusqu'au positionnement de l'extrémité inférieure du récipient échantillon **2** dans le champ du capteur **33.**

Alternativement dans le cas où le capteur **33** est un capteur linéaire de champ horizontal, l'unité **36** peut piloter le système de déplacement pour conférer au récipient un mouvement hélicoïdal combinant en continu la rotation autour de l'axe et la translation selon ledit axe, ce qui permet une acquisition d'une multitude d'images radiographiques ou de projections du récipient échantillon **2.**

Un appareil connu de tomographie assistée par ordinateur **30** tel que décrit ci-dessus est commercialisé par la société RX SOLUTIONS sous la dénomination commerciale EasyTom.

Un tel appareil de tomographie assistée par ordinateur **30** est relié au calculateur **38** qui dispose des images radiographiques du récipient échantillon **2** sous des angles de projection différents. Le calculateur **38** est programmé pour analyser les images radiographiques pour mettre en oeuvre la méthode de contrôle conforme à l'invention.

Il est à noter que le calculateur **38** est relié à un dispositif **39** pour connaître la position du récipient échantillon **2** dans le moule finisseur **14** identifié, selon le repère moule **X, Y, Z.** En d'autres termes, le calculateur **38** reçoit les informations concernant la position du récipient échantillon **2** dans le moule finisseur **14** identifié, selon le repère moule **X, Y, Z.**

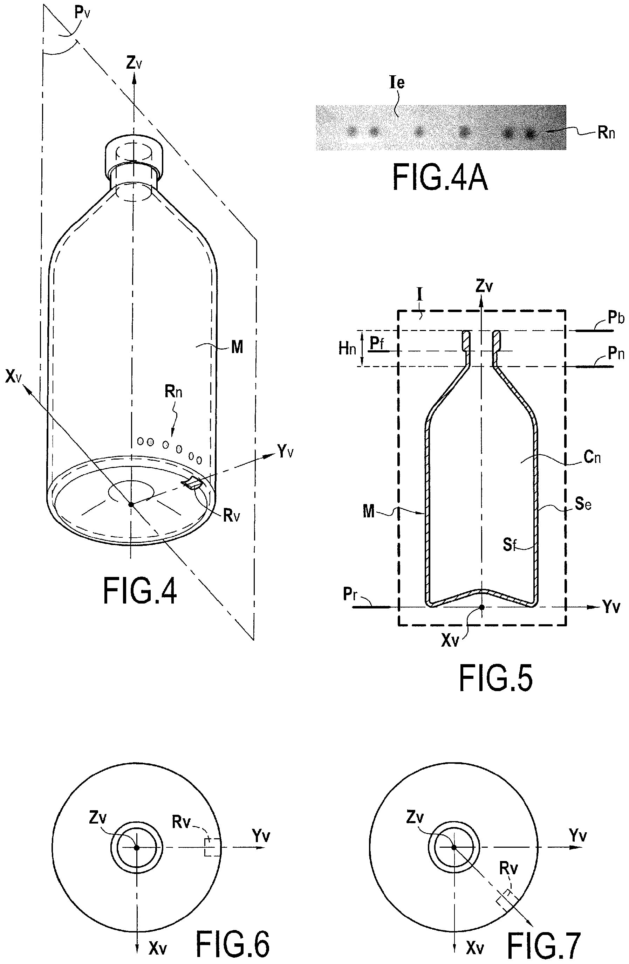

Le calculateur **38** est configurer ou programmé pour analyser les images radiographiques afin de construire dans un repère virtuel **Xv, Yv, Zv,** un modèle numérique tridimensionnel **M** du récipient échantillon **2** à partir des images radiographiques (**Fig. 4** et **5**). Comme les images radiographiques sont prises alors que le récipient échantillon **2** est vide, les images radiographiques **I** font apparaître uniquement la matière du récipient échantillon contrastée par rapport à l'air, dont l'atténuation est négligeable devant celle du verre constituant le récipient échantillon. Le modèle numérique tridimensionnel **M** présente ainsi une surface externe **Se** correspondant à la surface externe du récipient échantillon **2** et une surface interne **Sf** correspondant à la surface interne du récipient échantillon **2.**

La construction du modèle numérique tridimensionnel **M** est réalisée de toute manière appropriée connue de l'homme du métier. Typiquement, l'analyse des images radiographiques du récipient échantillon **2** vide permet de reconstruire un modèle numérique tridimensionnel du récipient échantillon sous la forme d'un ensemble de « voxels », c'est-à-dire de volumes unitaires dont la valeur est l'absorption de rayons X qu'ils créent, ce qui aboutit donc à une fonction de distribution volumique très similaire à une distribution de densité.

La réalisation d'un modèle numérique tridimensionnel est la façon - en termes mathématique, graphique et de structure de données - dont des objets tridimensionnels sont représentés et manipulés dans une mémoire d'ordinateur. Ce modèle numérique tridimensionnel est analysé pour mesurer des dimensions (longueurs, surfaces, épaisseurs, volumes). Le modèle numérique tridimensionnel peut rester volumique ou bien être transformé en modèle surfacique, c'est-à-dire dans lequel sont modélisées des surfaces séparant des volumes homogènes.

Dans les modélisations surfaciques, un objet est défini par son enveloppe, ses surfaces-frontières, ce qui permet d'appréhender les notions intérieur/extérieur, et les surfaces fermées définissent des volumes, auxquels on peut affecter par exemple une masse dès lors qu'on donne une masse volumique de la matière. Les surfaces se modélisent de plusieurs manières telles que par modélisation polygonale, par courbes ou surfaces paramétriques (cylindres, cônes, sphères, splines, ...) ou par subdivision de surfaces. A l'aide d'un maillage de polyèdres, par exemple des triangles, les surfaces des objets sont représentées par des ensembles de facettes planes connexes par leurs arêtes.

Une modélisation volumique consiste à baser la représentation sur des ensembles de volumes élémentaires identiques appelés Voxels.

Afin d'opérer des mesures de longueurs il existe plusieurs approches.

Dans une première méthode volumique, il est possible de parcourir un modèle volumique suivant une droite ou un faisceau de droites et déterminer les voxels de frontière matière / air.

Dans une deuxième méthode surfacique, il est possible de calculer un segment dont les extrémités sont les intersections d'une droite avec la surface d'un modèle surfacique. Les algorithmes résolvent assez bien les problèmes topologiques. Le point est unique. Enfin, une méthode mixte consiste à transformer le modèle volumique en modèle surfacique, puis à appliquer la deuxième méthode.

Dans la présente description, il doit être compris que la correspondance entre un élément du modèle numérique tridimensionnel **M** et un élément du récipient échantillon **2** signifie que l'élément du modèle numérique tridimensionnel **M** est la représentation virtuelle de l'élément du récipient échantillon **2.**

Le calculateur **38** est configuré ou programmé pour déterminer la position du modèle numérique tridimensionnel **M** par rapport à la position du récipient échantillon **2** dans le repère moule **X, Y, Z.** En d'autres termes, le modèle numérique tridimensionnel **M** correspondant au récipient échantillon **2** est analysé de manière à pouvoir être repéré dans une position connue par rapport à la position du récipient échantillon **2** dans le moule finisseur. Ainsi, il est possible pour toute région du modèle numérique tridimensionnel **M,** de connaître la position dans le moule finisseur, de la région du récipient échantillon **2** correspondante à cette région du modèle numérique tridimensionnel **M.**

Bien entendu, il peut être mis en oeuvre différentes méthodes de repérage du modèle numérique tridimensionnel **M** par rapport à la position du récipient échantillon dans son moule finisseur identifié.

Une première solution dite manuelle peut être envisagée consistant à considérer un relief de repérage **R** sur le récipient échantillon **2.** Par relief de repérage, il est compris en particulier, des reliefs portés par les récipients comme les reliefs de joint de moule ou des reliefs aménagés sur les récipients soit pour des fonctions esthétiques tels que les blasons ou les gravures décoratives soit pour des fonctions techniques (texte, code ou autre inscription de contenance, de numéro de moule, de numéro de lot, de marque, de modèle) ou soit pour des fonctions mécaniques tels que la contre-bague ou un filet de bouchon, un ergot ou une encoche de positionnement, les stries de contact fond, une garde étiquette. Dans l'exemple illustré à la **Fig. 3****,** le relief de repérage **R** sur le récipient échantillon **2** correspond à une encoche de positionnement aménagée au niveau du fond du récipient échantillon.

La position du relief de repérage **R** est connue dans le repère moule **X, Y, Z.**

Ce récipient échantillon **2** est positionné sur le porte-échantillon **31** de manière que son relief de repérage **R** soit positionné par rapport à un dispositif de repérage visuel ou mécanique du porte-échantillon **31.** Ainsi, comme l'appareil de tomographie assistée par ordinateur **30** connaît la position du dispositif de repérage visuel ou mécanique du porte-échantillon **31,** l'appareil construit le modèle numérique tridimensionnel **M** dans un repère virtuel **Xv, Yv, Zv,** connu par rapport au repère moule **X, Y, Z.** En d'autres termes, par rapport à la position réelle du récipient échantillon sur le porte-échantillon, le modèle numérique tridimensionnel **M** de ce récipient échantillon est créé permettant de localiser notamment la partie droite de la partie gauche de ce modèle correspondant respectivement aux parties droite et gauche du récipient échantillon, séparées par le plan de joint de moule.

Pour assurer le repérage du modèle numérique tridimensionnel **M,** une autre solution dite logicielle peut être envisagée consistant à choisir un relief de repérage **R** sur le récipient échantillon **2** dont la position est connue dans le repère moule **X, Y, Z** du moule finisseur dont est issu ledit récipient échantillon **2.** La méthode consiste ensuite à localiser sur le modèle numérique tridimensionnel **M,** le relief correspondant au relief de repérage **R** choisi sur le récipient échantillon **2,** et désigné relief de repérage virtuel **Rv** (**Fig. 4**). Il est ainsi possible de déterminer la position du relief de repérage virtuel **Rv** dans le repère virtuel **Xv, Yv, Zv,** pour en déduire la position du modèle numérique tridimensionnel **M** dans le repère moule **X, Y, Z** du moule finisseur identifié. Ainsi, pour toute région du modèle numérique tridimensionnel **M,** il est possible de connaître la position dans le moule finisseur, de la région du récipient échantillon **2** correspondante à cette région du modèle numérique tridimensionnel **M.** Le plan définit par les axes **Xv, Zv** du repère virtuel **Xv, Yv, Zv,** est le plan de joint virtuel **Pv** correspondant au plan de joint moule **P.**

Selon une variante avantageuse de réalisation, cette méthode de repérage consiste à construire le modèle numérique tridimensionnel **M** en prenant en compte le porte-échantillon **31** qui sert de plan de pose mécanique **Pp** pour le récipient échantillon **2.** Cette méthode consiste à positionner le modèle numérique tridimensionnel **M** du récipient posé sur son fond, sur un plan de référence **Pr** du repère virtuel considéré comme correspondant au plan de pose mécanique **Pp,** ce plan de référence **Pr** étant désigné aussi par un plan de pose virtuel dans la suite de la description.

Selon une caractéristique avantageuse de réalisation, le procédé consiste à positionner le modèle numérique tridimensionnel **M** du récipient échantillon sur le plan de référence ou plan de pose virtuel **Pr** de manière à ce que le modèle numérique tridimensionnel **M** du récipient échantillon, se trouve dressé en équilibre statique sur trois points de son fond au contact du plan de référence ou plan de pose virtuel **Pr.** Cette technique prend en compte la valeur d'une densité pour le matériau constitutif du récipient.

Il peut être choisi pour ce positionnement de simuler la gravité pour que le modèle numérique tridimensionnel **M** du récipient échantillon, se trouve dressé en équilibre statique sur trois points de son fond au contact du plan de référence ou plan de pose virtuel **Pr.**

Selon une autre variante avantageuse de réalisation, lorsque le procédé vise à déterminer la capacité du récipient, le procédé consiste à positionner le modèle numérique tridimensionnel **M** du récipient échantillon sur le plan de référence ou plan de pose virtuel **Pr** de manière à ce que par simulation de la gravité, le modèle numérique tridimensionnel du récipient rempli virtuellement jusqu'à un plan de niveau de remplissage par un liquide de densité déterminée, se trouve dressé en équilibre statique sur trois points de son fond au contact du plan de référence ou plan de pose virtuel **Pr.** Cette méthode de simulation permet d'approcher au mieux la réalité d'un récipient échantillon rempli d'un liquide et reposant sur un plan de pose.

Il est ainsi possible comme cela ressort de la **Fig. 5****,** de disposer d'un axe vertical virtuel **Zv** s'étendant perpendiculairement par rapport au plan de pose virtuel **Pr** du récipient échantillon sur le porte-échantillon. Tel qu'illustré aux **Fig. 6** et **7****,** le procédé consiste ensuite à assurer une rotation relative autour de l'axe vertical virtuel **Zv,** du modèle numérique tridimensionnel **M** afin d'amener le relief de repérage virtuel **Rv** dans une position correspondant à la position du relief de repérage **R** dans le repère moule du moule finisseur.

Dans l'analyse du modèle numérique tridimensionnel **M,** il avantageux comme expliqué précédemment de déterminer le plan de pose du récipient échantillon et d'utiliser ce plan comme base **Xv, Yv** du repère virtuel.

D'autres repérages sont avantageux. Par exemple on peut avoir à définir le sommet du modèle numérique tridimensionnel **M.** Ce sera le point le plus éloigné du plan de pose. On peut également déterminer un plan de surface de bague **Pb** comme :
- un plan passant par trois points de la surface de bague ;
- ou un plan moyen de la surface de bague ;
- ou un plan positionné en équilibre statique sur la surface de bague.

La méthode selon l'invention vise ensuite à analyser le modèle numérique tridimensionnel **M** pour déterminer au moins un indicateur de qualité **A** du récipient échantillon **2** en relation d'au moins une région du récipient échantillon. En d'autres termes, le calculateur **38** est programmé pour analyser le modèle numérique tridimensionnel **M** de manière à déterminer au moins un indicateur de qualité **A** du récipient échantillon **2** en relation d'au moins une région du récipient échantillon issu d'un moule finisseur identifié. Conformément à l'invention, l'analyse conduit à l'obtention d'au moins un indicateur de qualité **A** du récipient échantillon **2** permettant d'en déduire une information d'ajustement pour au moins un paramètre de contrôle du procédé de formage en relation du moule identifié du récipient échantillon **2.** En d'autres termes, cet indicateur de qualité **A** donne une information sur les corrections à apporter aux paramètres de contrôle du procédé de formage de l'installation de formage **3.**

Ces paramètres de contrôle visent les paramètres de contrôle du procédé de formage en relation en particulier du moule identifié du récipient échantillon **2.** Il est rappelé que le moule ébaucheur **13** et/ou le moule finisseur **14** d'où provient le récipient échantillon prélevé sont identifiés par un numéro de moule ou un numéro d'emplacement.

Selon une application préférée, la méthode selon l'invention vise à identifier le moule ébaucheur **13** et/ou le moule finisseur **14** d'où est issu le récipient échantillon **2** par un numéro de moule ou par un numéro d'emplacement et à mettre à disposition, ce numéro de moule ou ce numéro d'emplacement en relation de l'indicateur de qualité **A** du récipient échantillon. L'identification du moule et la mise à disposition du numéro de moule ou d'emplacement peuvent être réalisées de différentes manières.

Dans une utilisation totalement manuelle, l'opérateur prélève un récipient échantillon **2** en connaissant son numéro de moule ou d'emplacement. Une fois que le calculateur **38** a délivré la ou les valeurs du ou des indicateurs de qualité, l'opérateur peut agir sur le procédé en fonction du numéro de moule ou d'emplacement du récipient échantillon.

Dans les autres modes d'utilisation, la machine **1** selon l'invention comporte un système **40** fournissant au calculateur **38,** le numéro de moule ou d'emplacement du récipient échantillon **2.** Ce système **40** fourni le numéro de moule ou d'emplacement selon les diverses solutions suivantes.
a) Lors du chargement manuel du récipient échantillon sur le porte échantillon **31,** le système **40** est une interface de saisie permettant à l'opérateur de renseigner le numéro de moule ou d'emplacement du récipient échantillon.
b) En cas de chargement automatique d'une série de récipients échantillon, un ordre est préétabli avec la suite des numéros de moule ou d'emplacement des récipients échantillon successifs connue. Cette suite des numéros de moule ou d'emplacement des récipients échantillon est fournie par le système **40** au calculateur **38.** Alternativement l'organe de prélèvement et de chargement des récipients échantillon ou un système informatique de supervision, fournit par le système **40**, les numéros de moule ou d'emplacement de chaque récipient échantillon successif.
c) Il peut être prévu d'équiper la machine **1** d'un système **40** comportant un lecteur automatique **40a** par exemple optique, d'un relief porté par le récipient échantillon et indiquant le numéro de moule, ce système communiquant au calculateur **38** les numéros lus et éventuellement la table de correspondance avec les numéros d'emplacements.
d) Il peut également être prévu, que le système **40** pour fournir le numéro de moule ou d'emplacement soit réalisé par des moyens d'analyse mis en oeuvre par le calculateur **38** pour analyser le modèle numérique tridimensionnel **M** du récipient échantillon **2.** Cette analyse vise à rechercher sur le modèle numérique tridimensionnelle **M,** l'endroit d'un relief virtuel **Rn** indiquant le numéro de moule ou d'emplacement et correspondant au numéro de moule ou d'emplacement portée par le récipient échantillon **2.**

Dans l'exemple illustré à la **Fig.4****,** le modèle numérique tridimensionnel **M** du récipient échantillon **2** comporte en tant que relief virtuel **Rn** indiquant le numéro de moule ou d'emplacement, une suite d'empreintes réparties spatialement pour former un code.

Après avoir localisé ce relief virtuel **Rn,** les moyens d'analyse assurent la lecture de ce relief virtuel **Rn.** Selon une première méthode, le calculateur isole ledit relief virtuel de l'arrière-plan par soustraction d'une surface d'arrière-plan mise en correspondance par un algorithme de « best fit ». On peut obtenir comme illustrée à la **Fig. 4A****,** une image bidimensionnelle **Ie,** dans laquelle le code apparaît contrasté noir sur blanc ou blanc sur noir. Pour lire ce relief virtuel selon une deuxième méthode, le calculateur projette les épaisseurs de la paroi de la zone contenant le relief virtuel **Rn** pour obtenir une image d'épaisseur projetée. Dans cette image d'épaisseur **Ie,** qui est une image bidimensionnelle dans laquelle le niveau de gris représente l'épaisseur de verre projeté, le code apparaît contrasté noir sur blanc ou blanc sur noir. A partir des images bidimensionnelles obtenues, le code peut être alors analysé puis lu par un algorithme de traitement d'images connu par ailleurs, comprenant par exemple les étapes de segmentation et décodage ou d'OCR (Optical Character Recognition). Le code correspondant à ce relief virtuel est mis à la disposition du calculateur **38.**

La machine **1** conforme à l'invention délivre le ou les indicateurs de qualité **A** du récipient échantillon **2** sous toutes les formes possibles exploitables. A cet égard, la machine **1** conforme à l'invention comporte un système **41** pour délivrer au moins l'indicateur de qualité **A** du récipient échantillon en relation d'au moins une région du récipient échantillon. Par exemple, le système **41** pour délivrer au moins un indicateur de qualité **A** du récipient échantillon en relation d'au moins une région du récipient échantillon comporte un système d'affichage **42** pour l'indicateur de qualité en relation d'au moins une région du récipient échantillon, cet affichage étant accompagné de l'identité ou de l'identification du moule finisseur et/ou du moule ébaucheur identifié. A partir de cet indicateur de qualité **A,** un opérateur peut alors prendre les mesures correctrices adaptées en relation du moule finisseur identifié et/ou du moule ébaucheur identifié.

Selon un autre exemple de réalisation combiné ou non à l'exemple décrit ci-dessus, le système **41** pour délivrer au moins un indicateur de qualité du récipient échantillon en relation d'au moins une région du récipient échantillon comporte une connexion **43** pour transmettre au système de commande **23** de l'installation de formage **3,** l'indicateur de qualité **A** en relation de l'identité du moule finisseur. Ce système de commande **23** peut prendre automatiquement ou après validation, des mesures correctrices adaptées. Il est ainsi possible d'imaginer de mettre en place une table de correspondance entre les indicateurs de qualité **A** et les incidences sur les paramètres de contrôle du procédé de formage de l'installation de formage **3.**

De manière non limitative, l'indicateur de qualité **A** du récipient échantillon, permet de déduire une information d'ajustement pour au moins un paramètre de contrôle du procédé de formage des récipients pour les moules identifiés, parmi :
- le poids ou la forme de la paraison de verre chargée dans le moule ébaucheur identifié ;
- la position ou la vitesse de la paraison de verre à son chargement dans le moule ébaucheur identifié ;
- une synchronisation ou vitesse ou force dans le mouvement des mécanismes des poinçons de perçage, des moules identifiés, des transferts de l'ébauche, des pinces d'extraction;
- le refroidissement des moules identifiés ou d'un poinçon associé ;
- une pression de soufflage ou de pressage pour les moules identifiés ;
- le remplacement d'un moule identifié.

Selon une caractéristique avantageuse de l'invention, la méthode consiste à déterminer en tant qu'indicateur de qualité **A** du récipient échantillon, au moins un indicateur de qualité pris parmi la liste suivante à savoir :
- la répartition du verre du récipient échantillon ;
- la mesure d'au moins un volume du récipient échantillon prise parmi la capacité du récipient échantillon, le volume de l'enveloppe du récipient échantillon, le volume de verre du récipient échantillon et le volume de verre de la paraison chargée dans le moule ébaucheur identifié dont l'ébauche a été transférée dans le moule finisseur dont est issu le récipient échantillon **2** ;

- le rendu de reliefs aménagés sur le récipient échantillon ;
- la géométrie interne du goulot du récipient échantillon ;
- la planéité de la surface de la bague du récipient échantillon ;
- des diamètres extérieurs du corps du récipient échantillon.

La description qui suit vise à décrire la détermination de la répartition de verre en tant qu'indicateur de qualité **A** du récipient échantillon. Bien entendu, la répartition de verre du récipient échantillon **2** peut être mise en évidence selon divers paramètres ou caractéristiques déterminés à partir de l'analyse du modèle numérique tridimensionnel **M.**

Ainsi, la position du centre de masse est une caractéristique de la répartition de verre du récipient échantillon **2.**

La méthode selon l'invention vise à déterminer la position du centre de masse **Gv** du modèle numérique tridimensionnel **M** ou d'une portion dudit modèle, et à comparer cette position à une position de référence **Gr** du centre de masse.

Pour un récipient de révolution (par exemple de corps globalement conique ou cylindrique et sans gravure), on a un centre de masse théoriquement centré horizontalement sur l'axe de symétrie du récipient. Une méthode pour vérifier cette propriété est de calculer la projection parallèlement à l'axe vertical, sur un plan de coupe, de toute la matière du récipient. Le centre de masse de cette projection doit être centré à l'intersection de l'axe vertical et du plan de coupe.

Si le récipient n'est pas de simple révolution (forme générale asymétrique, présence de gravures), il est possible de mémoriser une position du centre de masse de référence apprise par exemple en analysant le modèle numérique tridimensionnel d'un récipient de référence dont la répartition de verre est correcte.

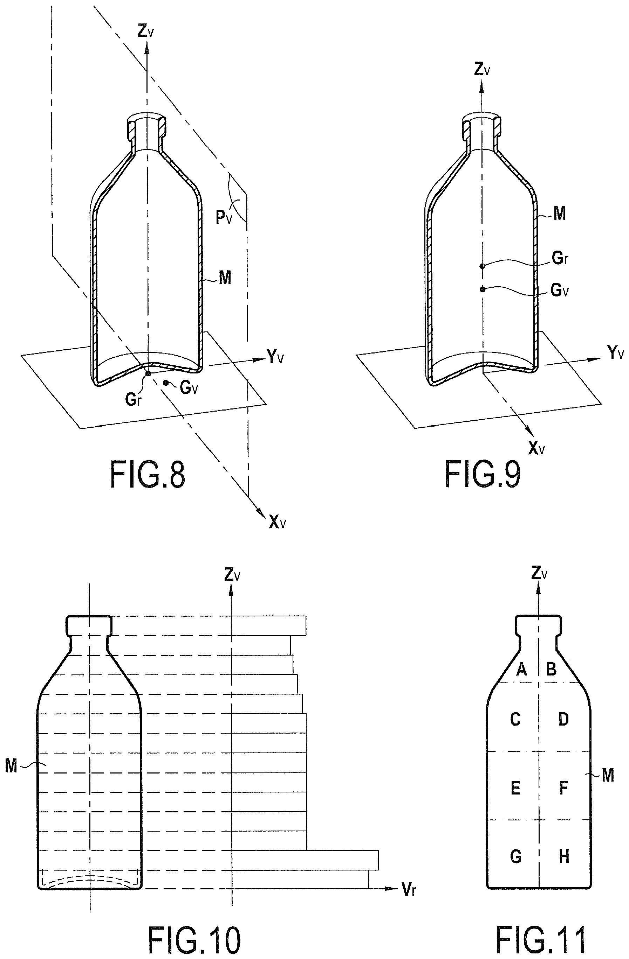

La **Fig. 8** illustre un exemple de réalisation dans lequel le centre de masse **Gv** du modèle numérique tridimensionnel **M** dans sa totalité est projeté dans un plan **Xv, Yv** du repère virtuel **Xv, Yv, Zv.** La position du centre de masse de référence **Gr** d'un récipient de référence dont la répartition de verre est correcte est calculée et projetée dans le plan **Xv, Yv** du repère virtuel **Xv, Yv, Zv.** Si les centres de masse **Gv** et **Gr** sont confondus, alors il peut en être conclu que la répartition de verre du récipient échantillon **2** est correcte. Dans l'exemple illustré, le centre de masse **Gv** du modèle numérique tridimensionnel **M** est décalé entre les directions positives **X** et **Y** c'est-à-dire vers l'avant du demi-moule droit.

La **Fig. 9** illustre un exemple de réalisation dans lequel la position du centre de masse de référence **Gr** d'un récipient de référence dont la répartition de verre est correcte est calculée et placée selon l'axe vertical **Zv** du repère virtuel **Xv, Yv, Zv** (pour un récipient de révolution par exemple). Le centre de masse **Gv** du modèle numérique tridimensionnel **M** dans sa totalité est calculé et éventuellement projeté sur l'axe vertical **Zv,** du repère virtuel **Xv, Yv, Zv.** Si les centres de masse **Gv** et **Gr** sont confondus, alors il peut en être conclu que la répartition de verre du récipient échantillon **2** est correcte. Dans l'exemple illustré, le centre de masse **Gv** du modèle numérique tridimensionnel **M** est décalé vers le bas.

Cette information sur le décalage du centre de masse donne une information pour ajuster les paramètres de contrôle du procédé de formage comme par exemple, la vitesse de la paraison, le moment de l'arrivée de la paraison, la lubrification des moules, etc.

Selon un autre exemple, l'épaisseur de la paroi de verre est aussi une caractéristique de la répartition de verre du récipient échantillon **2.**

Selon cet exemple, la méthode selon l'invention consiste à déterminer l'épaisseur de la paroi de verre sur au moins une région du récipient échantillon, en recherchant la position d'une zone avec une épaisseur supérieure à une valeur prédéfinie et/ou une épaisseur inférieure à une valeur prédéfinie, et/ou en recherchant dans les différentes zones la position et valeur des épaisseurs minimales ou maximales. La méthode vise ainsi à analyser le modèle numérique tridimensionnel **M** pour mesurer l'épaisseur entre la surface externe **Se** et la surface interne **Sf** sur une région ou la totalité de ce modèle numérique tridimensionnel **M.** Ces mesures sont comparées à des valeurs de seuil minimale et maximale permettant de déceler des zones trop minces ou trop épaisses et d'en mesurer l'étendue. Cette méthode permet bien entendu d'obtenir une cartographie de l'épaisseur du récipient échantillon **2.**

Il est également possible :
- de déterminer le volume de verre contenu dans au moins deux régions du modèle numérique tridimensionnel **M** divisé soit par un plan de section verticale contenant l'axe vertical virtuel **Zv** du modèle numérique tridimensionnel **M** ou soit par au moins un plan de section horizontale perpendiculaire audit axe vertical virtuel **Zv** ;
- et de comparer lesdits volumes à des valeurs de volume de référence et/ou entre plusieurs régions d'un même récipient échantillon, et/ou entre plusieurs récipients échantillons.

La **Fig. 10** illustre un exemple d'analyse du modèle numérique tridimensionnel **M** permettant de représenter la répartition selon l'axe vertical virtuel **Zv,** du volume **Vr** de verre prise selon des tranches parallèles perpendiculaires à l'axe vertical virtuel **Zv.** Bien entendu, cette répartition est comparée à une distribution de volumes obtenus à partir d'un modèle numérique tridimensionnel d'un récipient de référence.

La **Fig. 11** illustre un autre exemple d'analyse du modèle numérique tridimensionnel **M** permettant de représenter la répartition des volumes dans un plan contenant l'axe vertical virtuel **Zv.** Selon cet exemple, de part et d'autre de l'axe vertical virtuel **Zv,** sont représentés les volumes de verre situés selon quatre sections superposées à savoir **A-B, C-D, E-F** et **G-H.** Chacune de ces zones peuvent être comparées à des valeurs de volume de référence ou certaines de ces zones peuvent être comparées entre elles. Ainsi, les volumes des zones **C-D** peuvent être comparés aux volumes des zones **G-H** pour apprécier la répartition verticale du verre tandis qu'une comparaison globale ou deux à deux des zones **A, C, E** et **G** avec les zones **B, D, F** et **H** permet d'apprécier la répartition latérale du verre.

Cette information sur la répartition de l'épaisseur de la paroi de verre donne une information pour ajuster les paramètres de contrôle du procédé de formage comme par exemple, le conditions de chargement du moule finisseur (en agissant par exemple sur la position du déflecteur), la ventilation du moule ébaucheur, le graissage, etc.

La description qui suit vise à décrire en tant qu'indicateur de qualité **A** du récipient échantillon, au moins une mesure de volume du récipient échantillon **2** prise parmi, la capacité du récipient échantillon, le volume de l'enveloppe du récipient échantillon, le volume de verre du récipient échantillon et le volume de verre de la paraison chargée dans le moule ébaucheur identifié, dont il est rappelé que c'est le moule ébaucheur d'où l'ébauche a été transférée dans le moule finisseur identifié dont est issu le récipient échantillon **2.**

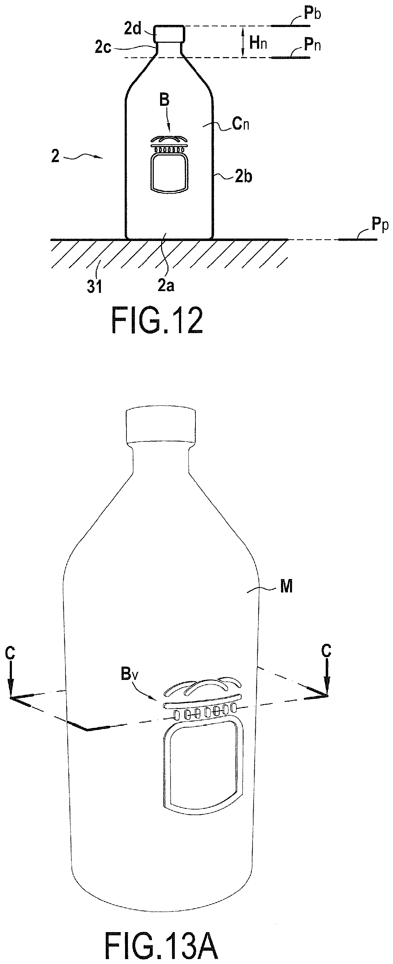

La **Fig. 12** permet d'illustrer la définition de la capacité des récipients **2** en verre. Un récipient **2** est un objet creux comportant classiquement, un fond **2a** à partir duquel s'élève un corps **2b** se prolongeant par un col **2c** terminé par une bague **2d** délimitant l'ouverture ou l'embouchure permettant de remplir ou de vider le récipient. La capacité ou la contenance du récipient **2** est le volume de liquide qu'il contient par la surface interne de sa paroi lorsque le récipient repose par son fond, en équilibre statique généralement par gravité sur un plan horizontal appelé plan de pose mécanique **Pp.**

La capacité ras-bord du récipient **1** correspond au volume de liquide remplissant le récipient jusqu'au plan dit de bague **Pb** passant par la bague **2d** du récipient, et plus précisément au niveau de la surface de la bague du récipient. La capacité nominale **Cn** du récipient **1** correspond au volume de liquide remplissant le récipient jusqu'à un plan de niveau **Pn** de remplissage du liquide, s'étendant parallèlement au plan de pose mécanique **Pp** et situé à une hauteur déterminée **Hn** du plan de bague **Pb.**

La détermination de la capacité du récipient échantillon **2,** passe par une étape d'analyse du modèle numérique tridimensionnel **M** du récipient échantillon **2** visant :
- à déterminer la surface interne **Sf** du modèle numérique tridimensionnel **M** du récipient échantillon **2** ;
- à positionner un plan de niveau de remplissage **Pn** sur le modèle numérique tridimensionnel **M** du récipient échantillon **2** parallèle au plan de pose et à une distance **Hn** du sommet du modèle numérique du récipient ;
- à mesurer par calcul, le volume interne du modèle numérique tridimensionnel **M** délimité par la surface interne **Sf** et par le plan de niveau de remplissage **Pn,** en sachant que cette mesure correspond à la capacité de remplissage **Cn** du récipient.

A partir du modèle numérique tridimensionnel **M,** le procédé consiste à déterminer la surface interne **Sf** du modèle numérique tridimensionnel comme correspondant à la surface interne du récipient échantillon **2.**

Le procédé consiste ensuite à positionner le plan de niveau de remplissage **Pn** de manière à fermer la surface interne du modèle numérique tridimensionnel **M** du récipient échantillon **2.** Ainsi, il est défini une surface fermée entourant ou enveloppant complètement le volume de remplissage du récipient.

Le procédé consiste ensuite à mesurer par calcul, le volume intérieur délimité par cette surface fermée à savoir par la surface interne **Sf** du modèle numérique tridimensionnel **M** et le plan de niveau de remplissage **Pn.** En effet, le volume intérieur délimité par cette surface fermée correspond au volume interne de remplissage du récipient échantillon jusqu'au niveau de remplissage.

Selon une caractéristique avantageuse de réalisation, le procédé consiste à positionner le modèle numérique tridimensionnel **M** du récipient échantillon posé sur son fond sur un plan de référence **Pr** de l'espace virtuel considéré comme horizontal par hypothèse. Comme ce plan de référence simule la pose du récipient échantillon sur un plan de pose mécanique, ce plan de référence **Pr** est désigné aussi par un plan de pose virtuel.

Comme expliqué précédemment, le plan de pose virtuel peut être la représentation du plan de pose mécanique dans l'espace virtuel.

Ensuite, le plan de niveau de remplissage **Pn** est positionné parallèlement au plan de référence ou plan de pose virtuel **Pr** à une distance **Hn** du sommet du modèle numérique tridimensionnel du récipient.

Selon une variante avantageuse de réalisation, le procédé consiste à positionner le modèle numérique tridimensionnel **M** du récipient échantillon sur le plan de référence ou plan de pose virtuel **Pr** de manière à ce que par simulation de la gravité, le modèle tridimensionnel du récipient, se trouve dressé en équilibre statique sur trois points de son fond au contact du plan de référence ou plan de pose virtuel **Pr.** Cette technique prend en compte la valeur d'une densité pour le matériau constitutif du récipient.

Selon une autre variante avantageuse de réalisation, le procédé consiste à positionner le modèle numérique tridimensionnel **M** du récipient sur le plan de référence ou plan de pose virtuel **Pr** de manière à ce que par simulation de la gravité, le modèle numérique tridimensionnel du récipient rempli virtuellement jusqu'au plan de niveau de remplissage par un liquide de densité déterminée, se trouve dressé en équilibre statique sur trois points de son fond au contact du plan de référence ou plan de pose virtuel **Pr.** Cette méthode de simulation permet d'approcher au mieux la réalité d'un récipient rempli d'un liquide et reposant sur un plan de pose définissant le plan de niveau de remplissage.

Dans le cas où le plan de niveau de remplissage **Pn** est positionné à une distance **Hn** du sommet du modèle numérique tridimensionnel **M** du récipient échantillon, le sommet du modèle numérique tridimensionnel **M** du récipient est déterminé comme le point appartenant au modèle numérique tridimensionnel, le plus éloigné du plan de référence ou plan de pose virtuel **Pr** ou comme le point d'intersection d'un plan de surface de bague **Pb** du modèle numérique tridimensionnel avec un axe de symétrie dudit modèle. Dans ce dernier cas, l'axe de symétrie est sensiblement orthogonal au plan de référence ou plan de pose virtuel **Pr** et le plan de surface de bague **Pb** est défini comme un plan passant par trois points de la surface de bague, ou un plan moyen de la surface de bague ou un plan positionné en équilibre statique sur la surface de bague. Bien entendu, le procédé selon l'invention peut être mis en oeuvre pour un récipient échantillon ne comportant pas un axe de symétrie.

Il découle de la description qui précède que pour mesurer la capacité à ras bord du récipient, le procédé consiste à positionner le plan de niveau de remplissage **Pn** à une distance **Hn** nulle du sommet du modèle numérique tridimensionnel.

Selon une variante du procédé, pour mesurer la capacité à ras bord du récipient, le procédé consiste à considérer que le plan de niveau de remplissage **Pn** est confondu avec plan de surface de bague **Pb.**

Dans le même sens, pour mesurer la capacité nominale **Cn** du récipient, le procédé consiste à positionner le plan de niveau de remplissage **Pn** à une distance nominale **Hn** du sommet du modèle numérique tridimensionnel.

Une autre mesure de volume du récipient échantillon **2** est le volume de l'enveloppe du récipient échantillon. Cette mesure permet de remonter au volume du moule finisseur identifié duquel est issu le récipient échantillon **2.** Pour la détermination du volume de l'enveloppe du récipient échantillon, la méthode consiste à :
- déterminer la surface externe **Se** du modèle numérique tridimensionnel **M** du récipient échantillon **2** ;
- déterminer un plan fermeture de volume comme étant le plan de surface de bague **Pb** ou le plan inférieur de joint de moule de bague ;
- et mesurer par calcul le volume intérieur délimité par la surface externe **Se** et le plan de fermeture comme étant le volume de l'enveloppe du récipient échantillon.

Selon une variante avantageuse, le volume du moule finisseur identifié duquel est issu le récipient échantillon **2** est déterminé en considérant le retreint du récipient échantillon dû au refroidissement qu'il a subi entre le moment de son moulage et le moment de l'acquisition des images radiographiques.

Selon une autre variante de cette mesure, il est possible de déterminer quelle partie du moule finisseur identifié est en cause, en partageant le volume mesuré, par le plan de joint de moule virtuel **Pv** en deux volumes de demi-moules. Ce faisant, pour davantage de précision, il est également envisageable de supprimer l'influence du volume contenu dans les moules de la bague et le volume contenu dans le moule de fond. En effet, les positions de tous les joints de moule et plans de joints étant déterminées dans le repère moule **X, Y, Z,** elles sont connues dans le repère virtuel **Xv, Yv, Zv,** selon l'invention. On peut donc retrancher du volume du la surface externe, les volumes contenus dans les moules de la bague et du fond.

Une autre mesure de volume du récipient échantillon **2** est le volume de verre du récipient échantillon. A cet effet, la méthode consiste à déterminer le volume de la paroi du modèle numérique tridimensionnel **M** du récipient échantillon, correspondant au volume de la paroi en verre du récipient échantillon **2.** La méthode vise à déterminer la surface qui englobe complètement la paroi du modèle numérique tridimensionnel **M,** et qui comprend donc la surface interne **Sf** reliée au niveau de la bague par la surface de bague, à la surface externe **Se.** Ce volume est une première mesure utilisable du volume de verre du récipient échantillon **2.**

Une autre mesure de volume du récipient échantillon **2** est le volume réel de verre du récipient échantillon. Cette mesure prend en compte le manque de matière dans la paroi du récipient échantillon, qui se présente sous la forme de bulles. A cet effet, le procédé analyse le modèle numérique tridimensionnel **M** en recherchant des bulles correspondant à des manques de matière entre la surface interne **Sf** et la surface externe **Se.** Le procédé mesure les volumes desdites bulles, qui sont ensuite soustraits au volume de la paroi du modèle numérique tridimensionnel **M,** déterminé entre la surface interne **Sf** et la surface externe **Se.** Cette mesure de volume correspond au volume de verre de la paraison chargée dans le moule ébaucheur identifié dont l'ébauche a été transférée dans le moule finisseur dont est issu le récipient échantillon **2.** Les bulles prises en considération sont des bulles de dimension supérieure à un seuil. En effet, les bulles extrêmement fines et uniformément reparties dans la matière sont liées à l'affinage du verre dans le four. Il faudrait une très grande résolution au tomographe pour les voir, ce qui augmente le coût du matériel (nano-foyer et capteur résolu) et le coût d'usage en raison de la durée d'acquisition qui serait nécessaire avec le matériel disponible actuellement. Ces bulles d'affinage étant présentes dans la paraison ne sont pas à prendre en compte pour le calcul de volume de paraison à partir du volume du récipient. En revanche, les bulles de dimensions supérieures à un seuil donné, qui se trouvent visibles avec un tomographe à simple micro-foyer, sont créés dans les canaux de délivre ou durant le chargement voire durant le perçage d'ébauche pour les plus grosses. Il convient donc de soustraire leur volume au volume du récipient afin de calculer le volume de paraison à partir du volume réel du récipient.

La présence, les dimensions et position des bulles ou bouillons de chargement ou de soufflage constituent un critère de qualité du récipient échantillon en relation avec les paramètres du procédé tels que le formage des paraisons (la température du verre trop froide près du plongeur), les conditions de chargement de la paraison dans l'ébauche, la ventilation du moule ébaucheur et poinçon (trop chauds) et les autres conditions de perçage de l'ébauche.

Selon une caractéristique avantageuse d'exploitation des mesures des volumes du récipient échantillon, la méthode selon l'invention consiste à :
- considérer comme étant une mesure du volume de la paraison chargé dans le moule ébaucheur, le volume de verre du modèle numérique tridimensionnel **M,** avec la prise en compte ou non des manques de matière ;
- considérer le volume intérieur délimité par la surface externe **Se** du modèle numérique tridimensionnel **M** et un plan de fermeture comme étant une mesure du volume interne du moule finisseur identifié ;
- considérer le volume délimité par la surface interne **Sf** du modèle numérique tridimensionnel et un plan de niveau de remplissage **Pn** comme étant une mesure de la capacité du récipient échantillon ;
- déduire des mesures de la capacité du récipient échantillon et du volume interne du moule finisseur, le volume de la paraison à charger dans le moule ébaucheur dont est issu le récipient échantillon **2** ;
- et décider lorsque la capacité du récipient échantillon n'est pas conforme, de modifier le poids de la paraison pour au moins le moule ébaucheur dont est issu le récipient échantillon ou de remplacer le moule finisseur.

Bien entendu, l'une et/ou l'autre des mesures des volumes du récipient échantillon, permettent de déduire une information d'ajustement pour divers autres paramètres de contrôle du procédé de formage en relation du moule du récipient échantillon. La mesure de la capacité peut conduire à modifier par exemple le dispositif d'extraction du moule finisseur. La mesure du volume de la paraison peut servir à régler la source de paraison et la coupe ciseaux. La mesure du volume interne du moule finisseur identifié peut permettre d'identifier une usure anormale liée à des paramètres de graissage (fréquence, dosage).

La description qui suit vise à décrire en tant qu'indicateur de qualité **A** du récipient échantillon, le rendu de reliefs **B** aménagés sur le récipient échantillon **2.**

Par relief **B,** il est compris en particulier, des reliefs portés par la surface externe des récipients comme les reliefs de joint de moule ou des reliefs aménagés sur les récipients soit pour des fonctions esthétiques tels que les blasons ou les gravures décoratives soit pour des fonctions techniques (texte, code ou autre inscription de contenance, de numéro de moule, de numéro de lot, de marque, de modèle) ou soit pour des fonctions mécaniques tel que la contre-bague ou filet de bouchon, ergot ou encoche de positionnement, stries de contact fond, garde étiquette.

La méthode selon l'invention vise à inspecter le ou les reliefs **B** dont on souhaite contrôler le rendu ou l'aspect, en contrôlant en particulier leurs caractéristiques géométriques. Dans l'exemple illustré à la **Fig. 12****,** le relief **B** sur le récipient échantillon **2** correspond à un blason aménagé au niveau de l'épaule sur la surface externe du récipient échantillon. Bien entendu, il peut être choisi d'inspecter partiellement ou en totalité, un ou plusieurs des reliefs présents sur le récipient échantillon **2.**

La méthode consiste à repérer sur le modèle numérique tridimensionnel **M** tel qu'illustré à la **Fig. 13A** le relief virtuel **Bv** correspondant au relief **B** du récipient échantillon **2.** Toutes les méthodes de localisation peuvent être mises en oeuvre en sachant que cette localisation est d'autant plus facilitée par le fait que comme expliqué précédemment, la position du modèle numérique tridimensionnel **M,** est connue dans le repère virtuel dont la relation avec le repère moule est aussi connue.

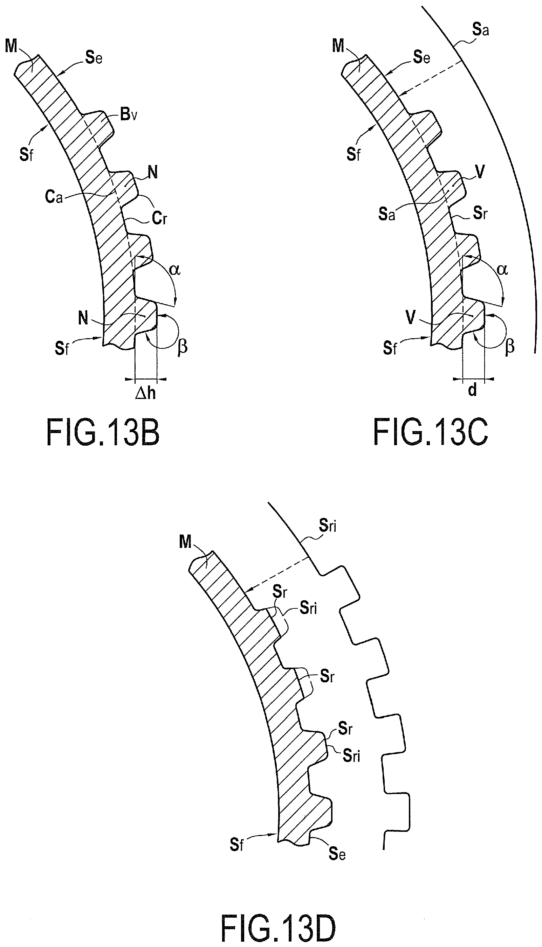

Pour la détermination du rendu d'un relief **B,** plusieurs méthodes sont possibles en considérant que le rendu du relief virtuel correspond au rendu du relief porté par le récipient échantillon. Selon l'exemple illustré plus précisément à la **Fig. 13B****,** la méthode selon l'invention consiste à :
- positionner au moins un plan de coupe **C-C** sur le modèle numérique tridimensionnel **M** du récipient échantillon de manière qu'il sectionne au moins une partie dudit relief virtuel **Bv** ;
- déterminer dans le plan de coupe **C-C,** la courbe représentative **Cr** de la section du relief virtuel;
- superposer au moins partiellement à la courbe représentative **Cr,** une courbe d'altitude zéro **Ca** correspondant à la courbe de la surface externe **Se** du récipient échantillon dépourvu dudit relief;
- et comparer la courbe représentative **Cr** à la courbe d'altitude zéro **Ca,** en calculant un critère de rendu du relief **B** pouvant se présenter sous différentes grandeurs.

Par exemple, comme critère de rendu du relief **B,** il peut être pris une distance entre la courbe d'altitude zéro **Ca** et la courbe représentative **Cr.** Il peut aussi être pris un écart de pente à une position donnée entre la courbe d'altitude zéro **Ca** et la courbe représentative **Cr** ou soit une variation de la pente de la courbe représentative **Cr.** La **Fig. 13B** illustre sous la forme d'un angle alpha, l'écart de pente à une position donnée entre la courbe d'altitude zéro **Ca** et la courbe représentative **Cr** et par un angle béta, une variation de la pente de la courbe représentative **Cr.** L'aire **N** délimitée par les courbes d'altitude zéro **Ca** et représentative **Cr** peut aussi être pris comme critère de rendu du relief **B.**

Il est à noter que cette variante de réalisation est avantageuse dans le cas où le relief à une fonction technique et dont la position est bien entendu connue. Cette méthode consiste à :
- sélectionner sur le modèle numérique tridimensionnel **M,** un relief virtuel correspondant à un relief à fonction technique dont la position est connue ;
- positionner sur le modèle numérique tridimensionnel **M,** un plan de section de manière qu'il coupe ledit relief virtuel dans un plan de section correspondant à un plan de conception ou de définition normative portant des indications de tolérance du relief à fonction technique ;
- obtenir une courbe représentative **Cr** de la section du relief ;
- mesurer sur cette courbe représentative, un rayon de courbure et/ou un angle, une longueur, une distance à une courbe **Ca** d'altitude zéro ;
- comparer ces mesures aux indications de tolérances du relief.

Pour la détermination du rendu des reliefs, il peut être envisagé, comme illustré à la **Fig. 13C****,** une autre méthode consistant à :
- déterminer la surface représentative **Sr** du relief comme une portion de surface externe du modèle numérique tridimensionnel **M** dans la zone d'intérêt contenant au moins une partie du relief virtuel correspondant au relief ;
- superposer au moins partiellement à la surface externe **Se** de la zone d'intérêt du relief virtuel, une surface d'altitude zéro **Sa** représentant la surface de la zone d'intérêt dépourvu dudit relief ;
- et comparer la surface représentative **Sr** à la surface d'altitude zéro **Sa,** en calculant un critère de rendu du relief **B** pouvant se présenter sous différentes des grandeurs comme ceux décrits ci-dessus. Ainsi, comme critère de rendu du relief, il peut être choisi au moins une des grandeurs suivantes :
   - une distance **d** entre la surface d'altitude zéro **Sa** et la surface représentative **Sr** ;
   - l'écart de pente α à une position donnée entre la surface d'altitude zéro **Sa** et la surface représentative **Sr** ;
   - une variation β des pentes de la surface d'altitude représentative **Sr** ;
   - des volumes **V** délimités par la surface d'altitude zéro **Sa** et la surface représentative **Sr.**

Pour la détermination du rendu des reliefs, il peut être envisagé une autre méthode comme illustré à la **Fig. 13D****,** consistant à :
- déterminer la surface représentative **Sr** du relief comme une portion de surface externe **Se** du modèle numérique tridimensionnel dans la zone d'intérêt contenant au moins une partie du relief virtuel correspondant au relief ;
- superposer au moins partiellement à la surface externe **Se** de la zone d'intérêt, une surface de relief théorique **Sri** représentant la surface de la zone d'intérêt si le relief est rendu correctement ;
- comparer la surface représentative **Sr** à la surface théorique **Sri,** en calculant comme critère de rendu du relief, au moins une des grandeurs suivantes :
   - une distance entre la surface représentative **Sr** et la surface théorique **Sri** ;
   - un écart de pente à une position donnée entre la surface représentative **Sr** et la surface théorique **Sri** ;
   - des volumes délimités par la surface représentative **Sr** et la surface théorique **Sri.**

L'une et/ou l'autre de ces grandeurs sont comparées par exemple, à des valeurs de référence pour déterminer la qualité de rendu de ces reliefs **B** pour permettre d'en déduire une information d'ajustement pour au moins un paramètre de contrôle du procédé de formage en relation du moule finisseur identifié du récipient échantillon. Typiquement, il est possible pour améliorer un rendu d'un relief, d'agir généralement sur l'étape de formage final dans le moule finisseur, en modifiant la ventilation du moule, ou le moment de soufflage (la durée de l'étirement) la durée de soufflage, l'entretien du moule finisseur, la mise au vide des évents.

La description qui suit vise à décrire en tant qu'indicateur de qualité **A** du récipient échantillon, la géométrie interne de goulot. Comme expliqué précédemment cette géométrie est définie par les valeurs de diamètres intérieurs du goulot à différentes hauteurs, voire sur toute la hauteur.

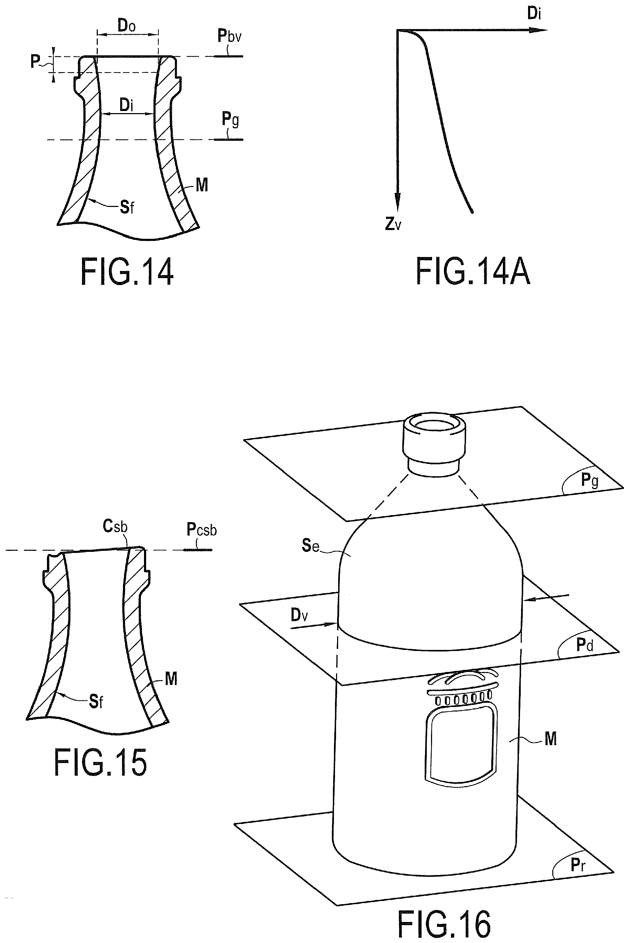

Selon l'invention, la méthode consiste comme illustré à la **Fig. 14****,** à déterminer dans le modèle numérique tridimensionnel **M,** la surface interne **Sf** correspondant au moins au goulot du récipient échantillon. La méthode consiste à choisir un plan de coupe **Pg** par exemple parallèle au plan de pose **Pr** du modèle (**Fig. 16**), et coupant à une hauteur donnée, le goulot du modèle numérique tridimensionnel. Il est alors possible de mesurer plusieurs diamètres de 0 à 360° dans ce plan de coupe. La méthode consiste à mesurer dans ce plan, plusieurs diamètres de la surface interne et à déterminer au moins le minimum et/ou le maximum dans le plan de coupe.

Il est aussi possible de déterminer la surface de la bague du modèle numérique tridimensionnel afin de déterminer le plan de la surface de bague **Pbv** du modèle comme expliqué précédemment. Ainsi, il est possible de déterminer le diamètre à l'ouverture **Do** (ou embouchure), par exemple à une distance **p**=5 mm sous l'embouchure en positionnant un plan de coupe 5 mm sous la surface de bague.

On peut aussi déterminer les diamètres sur toute la hauteur du goulot, en parcourant le goulot de la surface de bague (ou plan de surface de bague **Pbv**) jusqu'au bas du goulot par un plan de coupe parallèle au plan de pose **Pr** ou au plan de surface bague **Pbv** en mesurant plusieurs diamètres de 0 à 360° dans chacun de ces plans de coupe. Il est possible de déterminer par exemple le diamètre minimum sur les 360° pour chaque plan de coupe, et de considérer cette valeur de diamètre en fonction de la profondeur du plan de coupe de manière à obtenir le profil interne ou de débouchage. La figure **14A** donne à titre d'exemple le profil interne mesuré c'est-à-dire l'évolution des mesures du diamètre minimum interne **Di** selon l'axe vertical **Zv** sur toute la hauteur du goulot.

Alternativement, pour mesurer le « diamètre à l'ouverture », qui est spécifié par une tolérance de diamètre minimal et maximal, par exemple un intervalle de tolérance de 18 mm +/- 0.5, sur une profondeur donnée à partir de la surface de bague, par exemple 5 mm, il est possible de positionner de manière virtuelle, une première surface cylindrique de hauteur 5 mm, de diamètre maximum s'inscrivant dans la surface interne modélisée du goulot, et de même une seconde surface cylindrique de hauteur 5 mm, de diamètre minimum contenant la surface interne modélisée, et de considérer comme mesures du diamètre à l'ouverture du récipient échantillon, les diamètres des surfaces cylindriques inscrite et exinscrite, qui sont respectivement comparés aux tolérances.

Il est aussi possible de déterminer un diamètre minimum sur toute la hauteur de la surface interne du goulot pour vérifier le diamètre de brochage.

Le diamètre à l'ouverture, le diamètre de brochage, le profil interne du goulot, sont reliés à des paramètres du procédé de formage tels que la température des paraisons, celles des poinçons et moules ébaucheurs, la géométrie des moules de bague à l'ébaucheur, les « timing » de compression, de perçage et de soufflage.

La description qui suit vise à décrire en tant qu'indicateur de qualité **A** du récipient échantillon, la mesure de planéité de la surface de bague. La mesure de planéité de la surface de bague réalisée sur le modèle numérique tridimensionnel **M**, peut être réalisée de diverses manières.

Comme illustré à la **Fig. 15****,** une méthode consiste à déterminer une surface annulaire **Csb** représentative de la surface de la bague. Ladite surface est en théorie un anneau plan ou un tore parfait, mais il existe d'autres profils. On peut alors positionner un plan de référence **Pcsb** de surface de bague et analyser les écarts entre la surface représentative de la bague et ledit plan. On mesure et analyse les torsions de la surface transversalement et/ou tangentiellement. Ces torsions peuvent être des angles ou des courbures de la surface. On peut alternativement déterminer et mesurer les écarts entre ladite courbe tridimensionnelle fermée à un plan de référence positionné de différentes manières comme expliqué plus loin. Il a déjà été expliqué des méthodes pour mesurer des écarts entre des surfaces. Ainsi la comparaison de la surface représentative avec un plan consiste à mesurer des distances entre points de surfaces et/ou des volumes délimités par les surfaces. Dans ce cas par exemple, si la surface de bague est correcte, le volume entre cette surface et le plan de référence de surface de bague doit être nul.

Selon une autre variante, on détermine une courbe tridimensionnelle représentative de la surface de bague. Cette courbe est par exemple l'ensemble des points les plus hauts par rapport au plan de pose virtuel **Pr,** détectés sur toute la périphérie de la bague. Il peut s'agir aussi des points de jonctions entre la surface interne **Sf** et la surface externe **Se** du modèle. On peut déterminer et mesurer les écarts entre ladite courbe tridimensionnelle fermée représentative de la surface de bague et un plan de référence positionné de différentes manières comme expliqué plus loin. La mesure des écarts entre la courbe représentative et le plan de référence consiste par exemple à mesurer des distances entre des points de la courbe et des points correspondants du plan de référence de surface de bague. Ces distances sont par exemple selon l'axe **Zv.**

Le plan de référence peut être, le plan de surface de bague comme expliqué précédemment c'est-à-dire soit :
I. un plan passant par trois points de la surface de la bague ; on peut trouver un algorithme itératif simulant la pose d'un plan dans une position d'équilibre statique sur la courbe représentant la surface de bague ;
II. ou bien un plan moyen de la surface de bague, qui est par exemple le plan passant au mieux selon une fonction mathématique de distance par le point de la surface fermée.

Le critère de planéité peut être défini également par la courbure de la courbe représentative qui normalement est nulle (rayon de courbure infini).

Une autre méthode consiste à utiliser les coordonnées cylindriques (r, Z, θ : rayon r, hauteur Z, angle θ) avec l'axe vertical z correspondant à l'axe du goulot ou de la bague. Les défauts de planéité de la surface de bague sont souvent distingués en au moins deux types. Les défauts de type « manque de verre » sont liés à des problèmes de remplissage du moule de bague par le verre fondu lors du chargement de la paraison dans le moule finisseur. Ils se caractérisent par des écarts de hauteur (Δz) qui s'étendent sur une faible amplitude angulaire (Δθ) autour de la direction de l'axe vertical. Les défauts de type « bague voilée » sont des écarts de hauteur généralement moins marqués, qui s'étendent sur une plus grande amplitude angulaire autour de l'axe théorique, mais sont néanmoins des défauts gênants, souvent dus à des affaissements, à des problèmes mécaniques lors du transfert de l'extraction des articles du moule, ou à des problèmes thermiques de température de verre et de refroidissement. Mesurer la planéité de bague revient à déterminer des écarts entre la surface de bague et un plan.

Il apparaît ainsi que la mesure de planéité de la surface de bague est un indicateur de qualité pouvant être reliés à des paramètres du procédé de formage. Par exemple, un défaut de type non-rendu, correspond soit à un volume (ou poids) de paraison insuffisant pour remplir le moule ébaucheur, ou une pression insuffisante du poinçon en pressé-soufflé, ou une pression de soufflage insuffisante, ou une mauvaise compression.

La description qui suit vise à décrire en tant qu'indicateur de qualité **A** du récipient échantillon, des diamètres extérieurs du corps du récipient échantillon.

Selon l'invention, la méthode consiste comme illustré à la **Fig. 16**, à déterminer dans le modèle numérique tridimensionnel **M**, la surface externe **Se** correspondant à au moins la partie du récipient échantillon pour laquelle un diamètre extérieur est à mesurer. La méthode consiste à choisir à une hauteur donnée un plan de coupe **Pd** par exemple parallèle au plan de pose virtuel **Pr** du modèle, et à mesurer plusieurs diamètres **Dv** de 0 à 360° dans ce plan de coupe par rapport à la surface externe **Se**. Bien entendu, il peut être prévu de positionner le plan de coupe à différentes hauteurs du corps du récipient échantillon au niveau desquelles le diamètre extérieur est à mesurer. Le procédé consiste à comparer ces mesures à des valeurs de référence.

La mesure des diamètres extérieurs du corps du récipient échantillon est un indicateur de qualité pouvant être relié à des paramètres du procédé de formage tels que le refroidissement des moules, l'entretien des moules, les durée entre ouverture de moule et extraction etc.

Il est à noter que la machine **1** selon l'invention permet également de déterminer divers autres indicateurs de qualité du récipient échantillon. A partir de l'analyse du modèle numérique tridimensionnel **M**, il est possible de mesurer :
- la verticalité du corps, du col ou globale du récipient échantillon ;
- à autant de hauteurs qu'on le souhaite, des diamètres externes du corps, leur minimum et maximum et l'ovalisation du récipient échantillon ;
- la hauteur des récipients échantillon ;
- l'inclinaison de la bague par rapport au fond du récipient échantillon ;
- l'orientation de la bague par rapport au corps du récipient échantillon ;
- la qualité de jointure des moules (à partir des bavures laissées aux joints de moule) ;
- la courbure anormale de la paroi en creux ou bosse (gonflée) du récipient échantillon ;
- un affaissement de l'épaule du récipient échantillon.

Il ressort de la description qui précède que la machine **1** conforme à l'invention peut présenter différentes configurations en fonction du besoin des utilisateurs de connaître le ou les indicateurs de qualité du récipient échantillon.

Selon une configuration avantageuse, la machine selon l'invention est apte à déterminer en tant qu'indicateur de qualité du récipient échantillon, au moins un indicateur de qualité pris parmi la liste suivante à savoir :
- la répartition du verre du récipient échantillon ;
- la mesure d'au moins un volume du récipient échantillon prise parmi la capacité du récipient échantillon, le volume de l'enveloppe du récipient échantillon, le volume de verre du récipient échantillon et éventuellement le volume de verre de la paraison chargée dans le moule ébaucheur identifié dont l'ébauche a été transférée dans le moule finisseur dont est issu le récipient échantillon **2** ;
- et le rendu de reliefs aménagés sur le récipient échantillon.

Selon une autre configuration avantageuse, la machine selon l'invention est apte à déterminer en tant qu'indicateur de qualité du récipient échantillon, la mesure de la capacité du récipient échantillon, le volume de l'enveloppe du récipient échantillon, le volume de verre du récipient échantillon et éventuellement, le volume de verre de la paraison chargée dans le moule ébaucheur identifié dont l'ébauche a été transférée dans le moule finisseur dont est issu le récipient échantillon **2.**

Selon une autre configuration avantageuse, la machine selon l'invention est apte à déterminer en tant qu'indicateur de qualité du récipient échantillon, la répartition du verre du récipient échantillon, la mesure de la capacité du récipient échantillon, le volume de l'enveloppe du récipient échantillon, le volume de verre du récipient échantillon et éventuellement, le volume de verre de la paraison chargée dans le moule ébaucheur identifié dont l'ébauche a été transférée dans le moule finisseur dont est issu le récipient échantillon **2.**

Selon une autre configuration avantageuse, la machine selon l'invention est apte à déterminer en tant qu'indicateur de qualité du récipient échantillon, le rendu de reliefs aménagés sur le récipient échantillon, la répartition du verre du récipient échantillon, la mesure de la capacité du récipient échantillon, le volume de l'enveloppe du récipient échantillon, le volume de verre du récipient échantillon et éventuellement, le volume de verre de la paraison chargée dans le moule ébaucheur identifié dont l'ébauche a été transférée dans le moule finisseur dont est issu le récipient échantillon **2.**

Selon une autre configuration avantageuse, la machine selon l'invention est apte à déterminer en tant qu'indicateur de qualité du récipient échantillon, le rendu de reliefs aménagés sur le récipient échantillon, la répartition du verre du récipient échantillon, la mesure de la capacité du récipient échantillon, le volume de l'enveloppe du récipient échantillon, le volume de verre du récipient échantillon et éventuellement, le volume de verre de la paraison chargée dans le moule ébaucheur identifié dont l'ébauche a été transférée dans le moule finisseur dont est issu le récipient échantillon **2**, et au moins un autre critère pris parmi la liste suivante :
- la géométrie interne du goulot du récipient échantillon ;
- la planéité de la surface de la bague du récipient échantillon ;
- des diamètres extérieurs du corps du récipient échantillon.

Selon une autre configuration avantageuse, la machine selon l'invention est apte à déterminer en tant qu'indicateur de qualité du récipient échantillon, la répartition du verre du récipient échantillon, la mesure de la capacité du récipient échantillon et au moins un autre critère pris parmi la liste suivante :
- la géométrie interne du goulot du récipient échantillon ;
- la planéité de la surface de la bague du récipient échantillon ;
- des diamètres extérieurs du corps du récipient échantillon.

Selon une caractéristique avantageuse de réalisation, il peut être procédé à une opération de mise en correspondance du modèle numérique tridimensionnel du récipient échantillon avec un modèle numérique tridimensionnel de référence, représentant un récipient parfait, puis de déterminer des écarts de dimension en mesurant des distances entre des éléments de surface appartenant au modèle numérique de référence et des éléments de surface appartenant au modèle numérique tridimensionnel.

Il est à noter que la machine **1** conforme à l'invention peut comporter divers moyens de chargement et déchargement. Ces moyens peuvent comporter un convoyeur, un vérin linéaire avec une pince, un bras de robot, un chariot muni d'alvéoles recevant des séries de récipients échantillons à mesurer, etc.

Le calculateur **38** peut être relié à différents organes, comme un système de supervision, un système de contrôle et analyse statistique, un système de commande de l'installation de formage.

La machine **1** est installée de préférence à proximité de l'installation de fabrication comme représenté sur la **Fig. 1**, et les récipients échantillon sont prélevés au plus tard avant l'entrée de l'arche de recuisson de l'installation, ils sont généralement encore chaud. Si les récipients échantillon sont prélevés après leur passage dans l'arche de recuisson, alors le temps de réaction pour prendre en compte les indicateurs et modifier les paramètres du procédé est allongé de l'ordre de 30 mn à 1 heure, ce qui n'est pas favorable à la bonne exploitation des indicateurs de qualité.

Il est donc envisageable mais défavorable d'installer la machine **1** éloignée de la machine de fabrication, par exemple au secteur froid, après l'arche de recuisson, ou proche d'un service de qualité.

## Revendications

1. - Méthode pour contrôler un procédé de formage de récipients en verre (**2**) mettant en oeuvre une installation avec plusieurs sections de formage (**12**) distinctes dans chacune desquelles au moins une paraison de verre fondu (**18**) est en premier lieu mise en forme d'une ébauche dans au moins un moule ébaucheur (**13**), puis en second lieu mise en forme finale dans au moins un moule finisseur (**14**), **caractérisée en ce qu'**elle comporte les étapes suivantes :
- prélever un récipient dit échantillon issu d'un moule ébaucheur (**13**) identifié et d'un moule finisseur (**14**) identifié ;
- déposer le récipient échantillon (**2**) sur un porte-échantillon (**31**) d'un appareil de tomographie par rayons X assisté par ordinateur (**30**) ;
- acquérir au moyen de l'appareil de tomographie (**30**), plusieurs images radiographiques du récipient échantillon sous des angles de projection différents ;
- transmettre les images radiographiques à un calculateur (**38**) ;
- analyser les images radiographiques par le calculateur pour :
• construire dans un repère virtuel, un modèle numérique tridimensionnel (**M**) du récipient échantillon à partir des images radiographiques ;
- et analyser le modèle numérique tridimensionnel (**M**) pour déterminer au moins en tant qu'indicateur de qualité (**A**) du récipient échantillon, le rendu de reliefs (**B**) aménagés sur le récipient échantillon, permettant d'en déduire une information d'ajustement pour au moins un paramètre de contrôle du procédé de formage en relation du moule du récipient échantillon.

2. - Méthode selon la revendication précédente, **caractérisée en ce qu'**elle consiste à déterminer un indicateur de qualité (**A**) du récipient échantillon, permettant de déduire une information d'ajustement pour au moins un paramètre de contrôle du procédé de formage des récipients pour les moules identifiés, parmi :
- le poids ou la forme de la paraison de verre chargée dans le moule ébaucheur identifié ;
- la position ou la vitesse de la paraison de verre (**18**) à son chargement dans le moule ébaucheur identifié ;
- une synchronisation ou vitesse ou force dans le mouvement des mécanismes des poinçons de perçage, des moules identifiés, des transferts de l'ébauche, des pinces d'extraction;
- le refroidissement des moules identifiés ou d'un poinçon associé ;
- une pression de soufflage ou de pressage pour les moules identifiés ;
- le remplacement d'un moule identifié.

3. - Méthode selon l'une des revendications précédentes, **caractérisée en ce qu'**elle consiste à déterminer en tant qu'indicateur de qualité (**A**) du récipient échantillon (**2**), la répartition du verre du récipient échantillon.

4. - Méthode selon l'une des revendications précédentes, **caractérisée en ce qu'**elle consiste à déterminer en tant qu'indicateur de qualité (**A**) du récipient échantillon, au moins une mesure de volume du récipient échantillon prise parmi, la capacité (**Cn**) du récipient échantillon, le volume de l'enveloppe du récipient échantillon et le volume de verre du récipient échantillon.

5. - Méthode selon l'une des revendications précédentes, **caractérisée en ce qu'**elle consiste à déterminer en tant qu'indicateur de qualité (**A**) du récipient échantillon, la géométrie interne de goulot du récipient échantillon.

6. - Méthode selon l'une des revendications précédentes, **caractérisée en ce qu'**elle consiste à déterminer en tant qu'indicateur de qualité (**A**) du récipient échantillon, la planéité de la surface de bague du récipient échantillon.

7. - Méthode selon l'une des revendications précédentes, **caractérisée en ce qu'**elle consiste à déterminer en tant qu'indicateur de qualité (**A**) du récipient échantillon, des diamètres extérieurs du corps du récipient échantillon.

8. - Méthode selon la revendication 3, **caractérisée en ce qu'**elle consiste pour la détermination de la répartition de verre en tant qu'indicateur de qualité (**A**) du récipient échantillon, à déterminer l'épaisseur de la paroi de verre sur au moins une région du récipient échantillon (**2**), en recherchant dans cette région la position d'une zone avec une épaisseur supérieure à une valeur prédéfinie et/ou une épaisseur inférieure à une valeur prédéfinie, éventuellement en déterminant l'étendue de ladite zone, et/ou en recherchant la présence et la position de l'endroit de la paroi présentant le minimum ou le maximum d'épaisseur dans ladite région.

9. - Méthode selon la revendication 3, **caractérisée en ce qu'**elle consiste pour la détermination de la répartition de verre en tant qu'indicateur de qualité du récipient échantillon :
- à déterminer le volume de verre contenu dans au moins deux régions du modèle numérique tridimensionnel divisé soit par un plan de section verticale contenant l'axe vertical virtuel du modèle numérique tridimensionnel ou soit par un plan de section horizontale perpendiculaire audit axe vertical virtuel ;
- et à comparer lesdits volumes à des valeurs de volume de référence et/ou entre plusieurs régions d'un même récipient échantillon, et/ou entre plusieurs récipients échantillons.

10. - Méthode selon la revendication 1, **caractérisée en ce qu'**elle consiste pour la détermination du rendu de reliefs (**B**) aménagés sur le récipient échantillon en tant qu'indicateur de qualité du récipient échantillon à :
- positionner au moins un plan de coupe (**C-C**) sur le modèle numérique tridimensionnel (**M**) du récipient échantillon de manière qu'il sectionne au moins une partie d'un relief virtuel (**Br**) de la surface externe (**Se**) dudit modèle et correspondant au relief (**B**);
- déterminer dans le plan de coupe, la courbe représentative (**Cr**) de la section du relief virtuel (**Br**) ;
- superposer au moins partiellement à la courbe représentative (**Cr**), une courbe d'altitude zéro (**Ca**) représentant la courbe de la surface externe **(Se**) du récipient échantillon dépourvu dudit relief virtuel (**Br**) ;
- comparer la courbe représentative (**Cr**) à la courbe d'altitude zéro (**Ca**), en calculant comme critère de rendu du relief virtuel (**Br**) au moins une des grandeurs suivantes :
• une distance entre la courbe représentative (**Cr**) et la courbe d'altitude zéro (**Ca**) ;
• un écart de pente à une position donnée entre la courbe représentative (**Cr**) et la courbe d'altitude zéro (**Ca**) ;
• une variation de la pente de la courbe représentative (**Cr**) ;
• une aire délimitée par la courbe représentative (**Cr**) et la courbe d'altitude zéro (**Ca**).

11. - Méthode selon les revendications 1 ou 10, **caractérisée en ce qu'**elle consiste pour la détermination du rendu de reliefs (**B**) aménagés sur le récipient échantillon (**2**) en tant qu'indicateur de qualité (**A**) du récipient échantillon à :
- déterminer la surface représentative (**Sr**) du relief comme une portion de surface externe du modèle numérique tridimensionnel dans la zone d'intérêt contenant au moins une partie d'un relief virtuel correspondant au relief (**B**);
- superposer au moins partiellement à la surface externe de la zone d'intérêt, une surface d'altitude zéro (**Sa**) représentant la surface de la zone d'intérêt dépourvu dudit relief virtuel ;
- comparer la surface représentative (**Sr**) à la surface d'altitude zéro (**Sa**), en calculant comme critère de rendu du relief au moins une des grandeurs suivantes :
• une distance entre la surface d'altitude zéro (**Sa**) et la surface représentative (**Sr**) ;
• l'écart de pente à une position donnée entre la surface d'altitude zéro (**Sa**) et la surface représentative (**Sr**) ;
• une variation des pentes de la surface représentative (**Sr**) ;
• des volumes délimités par la surface d'altitude zéro (**Sa**) et la surface représentative (**Sr**).

12. - Méthode selon l'une des revendications 1, 10 ou 11, **caractérisée en ce qu'**elle consiste pour la détermination du rendu de reliefs aménagés sur le récipient échantillon en tant qu'indicateur de qualité du récipient échantillon à :
- déterminer la surface représentative d'un relief virtuel (**Sr**) comme une portion de surface externe du modèle numérique tridimensionnel dans la zone d'intérêt contenant au moins une partie du relief virtuel correspondant au relief du récipient échantillon ;
- superposer au moins partiellement à la surface externe de la zone d'intérêt, une surface de relief théorique (**Sri**) représentant la surface de la zone d'intérêt si le relief virtuel est rendu correctement ;
- comparer la surface représentative (**Sr**) à la surface théorique (**Sri**), en calculant comme critère de rendu du relief, au moins une des grandeurs suivantes :
• une distance entre la surface représentative (**Sr**) et la surface théorique (**Sri**) ;
• un écart de pente à une position donnée entre les surfaces (**Sr**) et (**Sri**) ;
• des volumes délimités par les surfaces (**Sr**) et (**Sri**).

13. - Méthode selon l'une des revendications 1, 10, 11 ou 12, **caractérisée en ce qu'**elle consiste pour la détermination du rendu de reliefs aménagés sur le récipient échantillon en tant qu'indicateur de qualité du récipient échantillon à :
- sélectionner sur le modèle numérique tridimensionnel (**M**), un relief virtuel correspondant à un relief à fonction technique dont la position est connue ;
- positionner un plan de section de manière qu'il coupe ledit relief dans un plan de section correspondant à un plan de conception ;
- obtenir une courbe représentative (**Cr**) de la section du relief virtuel ;
- mesurer sur cette courbe représentative, un rayon de courbure et/ou un angle, une longueur, une distance à une courbe (**Ca**) d'altitude zéro ;
- comparer la mesure à des valeurs de tolérance prédéfinies.

14. - Méthode selon la revendication précédente, **caractérisée en ce qu'**elle consiste à analyser le modèle numérique tridimensionnel (**M**) en recherchant des bulles correspondant à des manques de matière entre la surface interne (**Sf**) et la surface externe (**Se**), et à mesurer les volumes desdites bulles, qui sont ensuite soustraits au volume de la paroi du modèle numérique tridimensionnel (**M**), déterminé entre la surface interne (**Sf**) et la surface externe (**Se**), en vue d'obtenir un volume correspondant au volume de verre de la paraison chargée dans le moule ébaucheur identifié dont l'ébauche a été transférée dans le moule finisseur dont est issue le récipient échantillon (**2**).

15. - Méthode selon la revendication précédente, **caractérisée en ce qu'**elle consiste à :
- considérer comme étant une mesure du volume de la paraison chargé dans le moule ébaucheur, le volume de verre du modèle numérique tridimensionnel (**M**), avec la prise en compte ou non des manques de matière ;
- considérer le volume intérieur délimité par la surface externe du modèle numérique tridimensionnel (**M**) et un plan de fermeture comme étant une mesure du volume interne du moule finisseur ;
- considérer le volume délimité par la surface interne du modèle numérique tridimensionnel (**M**) et un plan de niveau de remplissage comme étant une mesure de la capacité (**Cn**) du récipient échantillon ;
- déduire des mesures de la capacité (**Cn**) du récipient échantillon et du volume interne du moule finisseur, le volume de la paraison à charger dans le moule ébaucheur dont est issu le récipient échantillon ;
- et décider lorsque la capacité du récipient échantillon n'est pas conforme, de modifier le poids de la paraison pour au moins le moule ébaucheur dont est issu le récipient échantillon ou de remplacer le moule finisseur.

16. - Méthode selon la revendication 5, **caractérisée en ce qu'**elle consiste, pour la détermination de la géométrie du goulot du récipient échantillon en tant qu'indicateur de qualité du récipient échantillon à :
- déterminer sur le modèle numérique tridimensionnel (**M**) la surface interne correspondant au moins à celle du goulot ;
- positionner au moins un plan de coupe (**Pg**) parallèle à un plan de pose virtuel (**Pr**) ;
- mesurer dans ce plan plusieurs diamètres de la surface interne et déterminer le minimum et/ou le maximum dans le plan de coupe ;
- à déterminer comme indicateur de la géométrie du goulot :
• le diamètre à l'ouverture ;
• et/ou le diamètre de brochage ;
• et/ou le profil interne du récipient échantillon.

17. - Méthode selon la revendication 6, **caractérisée en ce qu'**elle consiste, pour la détermination de la planéité de la surface de bague du récipient échantillon en tant qu'indicateur de qualité du récipient échantillon à :
- déterminer à partir du modèle numérique tridimensionnel (**M**), une courbe tridimensionnelle fermée ou une surface annulaire représentative de la surface de bague ;
- positionner un plan de référence de surface de bague en relation de la courbe tridimensionnelle fermée ou de la surface annulaire ;
- et mesurer les écarts entre le plan de référence et la courbe tridimensionnelle fermée ou la surface annulaire.

18. - Méthode selon la revendication 7, **caractérisée en ce qu'**elle consiste, pour la détermination des diamètres extérieurs du corps du récipient échantillon en tant qu'indicateur de qualité du récipient échantillon à :
- déterminer à partir du modèle numérique tridimensionnel (**M**), la surface externe (**Se**) correspondant au moins la partie du récipient échantillon pour laquelle un diamètre extérieur est à mesurer ;
- positionner un plan de coupe (**Pd**) parallèle au plan de pose virtuel (**Pr**) du modèle selon au moins une hauteur du récipient ;
- mesurer plusieurs diamètres dans ce plan de coupe par rapport à la surface externe et à comparer ces mesures à des valeurs de référence.

19. - Machine pour contrôler un procédé de formage de récipients en verre mettant en oeuvre une installation avec plusieurs sections de formage (**12**) distinctes dans chacune desquelles au moins une paraison de verre fondu (**18**) est en premier lieu mise en forme d'une ébauche dans au moins un moule ébaucheur(**13**), puis en second lieu mise en forme finale dans au moins un moule finisseur (**14**), **caractérisée en ce qu'**elle comporte :
- un appareil de tomographie par rayons X assisté par ordinateur (**30**), apte à réaliser plusieurs images radiographiques sous des angles de projection différents d'un récipient échantillon placé sur un porte-échantillon dudit appareil ;
- un calculateur (**38**) relié au dispositif (**39**) et à l'appareil de tomographie (**30**) et configuré pour analyser les images radiographiques pour :
• construire dans un repère virtuel, un modèle numérique tridimensionnel (**M**) du récipient échantillon à partir des images radiographiques ;
• analyser le modèle numérique tridimensionnel (**M**) pour déterminer au moins en tant qu'indicateur de qualité (**A**) du récipient échantillon, le rendu de reliefs (**B**) aménagés sur le récipient échantillon, permettant d'en déduire une information d'ajustement pour au moins un paramètre de contrôle du procédé de formage en relation du moule du récipient échantillon ;
- et un système (**41**) pour délivrer au moins l'indicateur de qualité (**A**) du récipient échantillon en relation d'au moins une région du récipient échantillon.

20. - Machine selon la revendication précédente, **caractérisée en ce que** le système (**41**) pour délivrer au moins l'indicateur de qualité du récipient échantillon en relation d'au moins une région du récipient échantillon comporte un système d'affichage (**42**) pour l'indicateur de qualité en relation de l'identité du moule finisseur.

21. **-** Machine selon la revendication précédente, **caractérisée en ce que** le système (**41**) pour délivrer au moins l'indicateur de qualité du récipient échantillon en relation d'au moins une région du récipient échantillon comporte une connexion (**43**) pour transmettre à un système de commande (**23**) de l'installation de formage, l'indicateur de qualité (**A**) en relation de l'identité du moule finisseur.

22. - Machine selon l'une des revendications 19 à 21, **caractérisée en ce qu'**elle comporte un système (**40**) fournissant au calculateur (**38**), le numéro de moule ou d'emplacement du récipient échantillon (**2**).

23. - Installation de formage de récipients en verre comportant plusieurs sections de formage (**12**) distinctes dans chacune desquelles au moins une paraison (**18**) de verre fondu est en premier lieu mise en forme d'une ébauche dans au moins un moule ébaucheur (**13**), puis en second lieu mise en forme finale dans au moins un moule finisseur (**14**), **caractérisée en ce qu'**elle comporte une machine (**21**) conforme à l'une des revendications 19 à 22, disposée à la sortie des moules finisseurs.

## Patentansprüche

1. Verfahren zur Steuerung eines Verfahrens zum Formen von Glasbehältern (2), bei welchem eine Anlage mit mehreren verschiedenen Formabschnitten (12) verwendet wird, wobei in jedem davon mindestens ein geschmolzener Glasposten (18) zuerst aus einem Rohling in mindestens einer Vorform (13) geformt wird, dann als zweites in mindestens einer Endform (14) endgeformt wird, **dadurch gekennzeichnet, dass** es die folgenden Schritte umfasst:
- Entnehmen eines Behälters, der als Probe bezeichnet wird und aus einer identifizierten Vorform (13) und aus einer identifizierten Endform (14) stammt;
- Anordnen des Probebehälters (2) auf einem Probenträger (31) einer computergestützten Röntgenstrahl-Tomografievorrichtung (30);
- Erfassen, mit Hilfe der Tomografievorrichtung (30), mehrerer Röntgenbilder des Probebehälters unter verschiedenen Projektionswinkeln;
- Übertragen der Röntgenbilder zu einem Rechner (38);
- Analysieren der Röntgenbilder durch den Rechner, um:
• in einer virtuellen Markierung ein dreidimensionales digitales Modell (M) des Probebehälters aus den Röntgenbildern zu konstruieren;
- und Analysieren des dreidimensionalen digitalen Modells (M), um mindestens als Qualitätsindikator (A) des Probebehälters die Darstellung der Reliefs (B) zu bestimmen die auf dem Probebehälter angeordnet sind, wodurch ermöglicht wird, daraus eine Information der Einstellung für mindestens einen Parameter zur Steuerung des Formverfahrens in Bezug auf die Form des Probebehälters abzuleiten.

2. Verfahren gemäß dem vorhergehenden Anspruch, **dadurch gekennzeichnet, dass** es darin besteht, einen Qualitätsindikator (A) des Probebehälters zu bestimmen, der ermöglicht, eine Information der Einstellung für mindestens einen Parameter zur Steuerung des Verfahrens zum Formen von Behältern für die identifizierten Formen abzuleiten, darunter:
- das Gewicht oder die Gestalt des Glaspostens, der in die identifizierte Vorform geladen wird;
- die Position oder die Geschwindigkeit des Glaspostens (18) bei seinem Laden in die identifizierte Vorform;
- eine Synchronisation oder Geschwindigkeit oder Kraft in der Bewegung der Mechanismen von Lochstempeln, identifizierten Formen, Transfers des Rohlings, Entnahmezangen;
- das Abkühlen der identifizierten Formen oder eines assoziierten Stempels;
- ein Blas- oder Pressdruck für die identifizierten Formen;
- das Ersetzen einer identifizierten Form.

3. Verfahren gemäß einem der vorhergehenden Ansprüche, **dadurch gekennzeichnet, dass** es daraus besteht, als Qualitätsindikator (A) des Probebehälters (2) die Verteilung des Glases des Probebehälters zu bestimmen.

4. Verfahren gemäß einem der vorhergehenden Ansprüche, **dadurch gekennzeichnet, dass** es daraus besteht, als Qualitätsindikator (A) des Probebehälters mindestens ein Maß des Volumens des Probebehälters zu bestimmen, ausgewählt aus der Kapazität (Cn) des Probebehälters, dem Volumen der Hüllkurve des Probebehälters und dem Volumen des Glases des Probebehälters.

5. Verfahren gemäß einem der vorhergehenden Ansprüche, **dadurch gekennzeichnet, dass** es daraus besteht, als Qualitätsindikator (A) des Probebehälters die innere Geometrie des Halses des Probebehälters zu bestimmen.

6. Verfahren gemäß einem der vorhergehenden Ansprüche, **dadurch gekennzeichnet, dass** es daraus besteht, als Qualitätsindikator (A) des Probebehälters die Ebenheit der Fläche des Rings des Probebehälters zu bestimmen.

7. Verfahren gemäß einem der vorhergehenden Ansprüche, **dadurch gekennzeichnet, dass** es daraus besteht, als Qualitätsindikator (A) des Probebehälters die Außendurchmesser des Körpers des Probebehälters zu bestimmen.

8. Verfahren gemäß Anspruch 3, **dadurch gekennzeichnet, dass** es zur Bestimmung der Verteilung des Glases als Qualitätsindikator (A) des Probebehälters daraus besteht, die Dicke der Glaswand in mindestens einer Zone des Probebehälters (2) zu bestimmen, indem in dieser Region die Position einer Zone mit einer Dicke, die größer ist als ein vorherbestimmter Wert, und/oder einer Dicke, die kleiner ist als ein vorherbestimmter Wert, gesucht wird, indem gegebenenfalls die Ausdehnung der Zone bestimmt wird, und/oder indem das Vorliegen und die Position des Ortes der Wand gesucht werden, welche die minimale oder die maximale Dicke in der Region aufweist.

9. Verfahren gemäß Anspruch 3, **dadurch gekennzeichnet, dass** es zur Bestimmung der Verteilung des Glases als Qualitätsindikator des Probebehälters daraus besteht:
- das Volumen des Glases zu bestimmen, das in mindestens zwei Regionen des dreidimensionalen digitalen Modells enthalten ist, geteilt entweder durch eine vertikale Schnittebene, welche die virtuelle vertikale Achse des dreidimensionalen digitalen Modells enthält, oder durch eine horizontale Schnittebene senkrecht zu der virtuellen vertikalen Achse;
- und die Volumina mit Referenzvolumenwerten und/oder zwischen mehreren Regionen desselben Probebehälters und/oder zwischen mehreren Probebehältern zu vergleichen.

10. Verfahren gemäß Anspruch 1, **dadurch gekennzeichnet, dass** es zur Bestimmung der Darstellung der Reliefs (B), die an dem Probebehälter angeordnet sind, als Qualitätsindikator des Probebehälters daraus besteht:
- mindestens eine Schnittebene (C-C) an dem dreidimensionalen digitalen Modell (M) des Probebehälters derart zu positionieren, dass sie mindestens einen Teil eines virtuellen Reliefs (Br) der Außenfläche (Se) des Modells und entsprechend dem Relief (B) schneidet;
- in der Schnittebene die repräsentative Kurve (Cr) der Sektion des virtuellen Reliefs (Br) zu bestimmen;
- mindestens teilweise über die repräsentative Kurve (Cr) eine Kurve mit der Höhe Null (Ca) zu legen, welche die Kurve der Außenfläche (Se) des Probebehälters ohne das virtuelle Relief (Br) darstellt:
- die repräsentative Kurve (Cr) mit der Kurve mit der Höhe Null (Ca) zu vergleichen, indem als Kriterium der Darstellung des virtuellen Reliefs (Br) mindestens eine der folgenden Größen berechnet wird:
• eine Distanz zwischen der repräsentativen Kurve (Cr) und der Kurve mit der Höhe Null (Ca);
• eine Abweichung der Steigung an einer gegebenen Position zwischen der repräsentativen Kurve (Cr) und der Kurve mit der Höhe Null (Ca);
• eine Variation der Steigung der repräsentativen Kurve (Cr);
• eine Fläche, die von der repräsentativen Kurve (Cr) und der Kurve mit der Höhe Null (Ca) begrenzt wird.

11. Verfahren gemäß den Ansprüchen 1 oder 10, **dadurch gekennzeichnet, dass** es zur Bestimmung der Darstellung der Reliefs (B), die an dem Probebehälter (2) angeordnet sind, als Qualitätsindikator (A) des Probebehälters daraus besteht:
- die repräsentative Fläche (Sr) des Reliefs als einen Abschnitt der Außenfläche des dreidimensionalen digitalen Modells in der Zone von Interesse zu bestimmen, die mindestens einen Teil eines virtuellen Reliefs entsprechend dem Relief (B) enthält;
- mindestens teilweise über die Außenfläche der Zone von Interesse eine Fläche mit der Höhe Null (Sa) zu legen, welche die Fläche der Zone von Interesse ohne das virtuelle Relief darstellt;
- die repräsentative Fläche (Sr) mit der Fläche mit der Höhe Null (Sa) zu vergleichen, indem als Kriterium der Darstellung des Reliefs mindestens eine der folgenden Größen berechnet wird:
• eine Distanz zwischen der Fläche mit der Höhe Null (Sa) und der repräsentativen Fläche (Sr);
• die Abweichung der Steigung an einer gegebenen Position zwischen der Fläche mit der Höhe Null (Sa) und der repräsentativen Fläche (Sr);
• eine Variation der Steigungen der repräsentativen Fläche (Sr);
• Volumina, die von der Fläche mit der Höhe Null (Sa) und der repräsentativen Fläche (Sr) begrenzt werden.

12. Verfahren gemäß einem der Ansprüche 1, 10 oder 11, **dadurch gekennzeichnet, dass** es zur Bestimmung der Darstellung der Reliefs, die an dem Probebehälter angeordnet sind, als Qualitätsindikator des Probebehälters daraus besteht:
- die repräsentative Fläche eines virtuellen Reliefs (Sr) als Abschnitt der Außenfläche des dreidimensionalen digitalen Modells in der Zone von Interesse zu bestimmen, die mindestens einen Teil des virtuellen Reliefs entsprechend dem Relief des Probebehälters enthält;,
- mindestens teilweise über die Außenfläche der Zone von Interesse eine theoretische Fläche des Reliefs (Sri) zu legen, welche die Fläche der Zone von Interesse darstellt, wenn das virtuelle Relief korrekt dargestellt wird;
- die repräsentative Fläche (Sr) mit der theoretischen Fläche (Sri) zu vergleichen, indem als Kriterium der Darstellung des Reliefs mindestens eine der folgenden Größen berechnet wird:
• eine Distanz zwischen der repräsentativen Fläche (Sr) und der theoretischen Fläche (Sri);
• eine Abweichung der Steigung an einer gegebenen Position zwischen den Flächen (Sr) und (Sri);
• Volumina, die von den Flächen (Sr) und (Sri) begrenzt werden.

13. Verfahren gemäß einem der Ansprüche 1, 10, 11 oder 12, **dadurch gekennzeichnet, dass** es zur Bestimmung der Darstellung der Reliefs, die an dem Probebehälter angeordnet sind, als Qualitätsindikator des Probebehälters daraus besteht:
- an dem dreidimensionalen digitalen Modell (M) ein virtuelles Relief entsprechend einem Relief mit einer technischen Funktion auszuwählen, dessen Position bekannt ist;
- eine Schnittebene derart zu positionieren, dass sie das Relief in einer Schnittebene entsprechend einer Konstruktionsebene schneidet;
- eine repräsentative Kurve (Cr) der Sektion des virtuellen Reliefs zu erhalten;
- an dieser repräsentativen Kurve einen Krümmungsradius und/oder einen Winkel, eine Länge, eine Distanz zu einer Kurve (Ca) mit der Höhe Null zu messen;
- das Maß mit vordefinierten Toleranzwerten zu vergleichen.

14. Verfahren gemäß dem vorhergehenden Anspruch, **dadurch gekennzeichnet, dass** es daraus besteht, das dreidimensionale digitale Modell (M) zu analysieren, indem Blasen, die Materialmängeln entsprechen, zwischen der Innenfläche (Sf) und der Außenfläche (Se) gesucht werden, und die Volumina der Blasen zu messen, die anschließend von dem Volumen der Wand des dreidimensionalen digitalen Modells (M) subtrahiert werden, das zwischen der Innenfläche (Sf) und der Außenfläche (Se) bestimmt wird, um ein Volumen zu erhalten, das dem Glasvolumen des Glaspostens entspricht, der in die identifizierte Vorform geladenen wird, wobei der Rohling in die Endform transferiert wurde, aus welcher der Probenbehälter (2) stammt.

15. Verfahren gemäß dem vorhergehenden Anspruch, **dadurch gekennzeichnet, dass** es daraus besteht:
- als ein Maß des Volumens des Postens, der in die Vorform geladen wird, das Glasvolumen des dreidimensionalen digitalen Modells (M) zu betrachten, wobei Materialfehler berücksichtigt werden oder nicht;
- das Innenvolumen, das von der Außenfläche des dreidimensionalen digitalen Modells (M) und einer Schließebene begrenzt wird, als ein Maß des Innenvolumens der Endform zu betrachten;
- das Volumen, das von der Innenfläche des dreidimensionalen digitalen Modells (M) und einer Ebene der Füllhöhe begrenzt wird, als ein Maß der Kapazität (Cn) des Probebehälters zu betrachten;
- von den Maßen der Kapazität (Cn) des Probebehälters und des Innenvolumens der Endform das Volumen des Postens abzuleiten, der in die Vorform zu laden ist, aus welcher der Probebehälter stammt;
- und zu entscheiden, wenn die Kapazität des Probebehälters nicht konform ist, das Gewicht des Postens für mindestens die Vorform zu modifizieren, aus welcher der Probebehälter stammt, oder die Endform zu ersetzen.

16. Verfahren gemäß Anspruch 5, **dadurch gekennzeichnet, dass** es zur Bestimmung der Geometrie des Halses des Probebehälters als Qualitätsindikator des Probebehälters daraus besteht:
- an dem dreidimensionalen digitalen Modell (M) die Innenfläche entsprechend mindestens jener des Halses zu bestimmen;
- mindestens eine Schnittebene (Pg) parallel zu einer virtuellen Auflageebene (Pr) zu positionieren;
- in dieser Ebene mehrere Durchmesser der Innenfläche zu messen und das Minimum und/oder das Maximum in der Schnittebene zu bestimmen;
- als Indikator der Geometrie des Halses zu bestimmen:
• den Durchmesser der Öffnung;
• und/oder den Heftungsdurchmesser;
• und/oder das Innenprofil des Probebehälters.

17. Verfahren gemäß Anspruch 6, **dadurch gekennzeichnet, dass** es zur Bestimmung der Ebenheit der Ringfläche des Probebehälters als Qualitätsindikator des Probebehälters daraus besteht:
- aus dem dreidimensionalen digitalen Modell (M) eine geschlossene dreidimensionale Kurve oder eine ringförmige Fläche zu bestimmen, die für die Ringfläche repräsentativ ist;
- eine Referenzebene der Ringfläche in Bezug auf die geschlossene dreidimensionale Kurve oder die ringförmige Fläche zu positionieren;
- und die Abweichungen zwischen der Referenzebene und der geschlossenen dreidimensionalen Kurve oder der ringförmigen Fläche zu messen.

18. Verfahren gemäß Anspruch 7, **dadurch gekennzeichnet, dass** es zur Bestimmung der Außendurchmesser des Körpers des Probebehälters als Qualitätsindikator des Probebehälters daraus besteht:
- aus dem dreidimensionalen digitalen Modell (M) die Außenfläche (Se) entsprechend mindestens dem Teil des Probebehälters zu bestimmen, für den ein Außendurchmesser zu berechnen ist;
- eine Schnittebene (Pd) parallel zur virtuellen Auflageebene (Pr) des Modells gemäß mindestens einer Höhe des Behälters zu positionieren;
- mehrere Durchmesser in dieser Schnittebene in Bezug auf die Außenfläche zu messen und diese Maße mit Referenzwerten zu vergleichen.

19. Maschine zur Steuerung eines Verfahrens zum Formen von Glasbehältern, bei dem eine Anlage mit mehreren verschiedenen Formabschnitten (12) verwendet wird, wobei in jedem davon mindestens ein geschmolzener Glasposten (18) zuerst aus einem Rohling in mindestens einer Vorform (13) geformt wird, dann als zweites in mindestens einer Endform (14) endgeformt wird, **dadurch gekennzeichnet, dass** sie umfasst:
- eine computergestützte Röntgenstrahl-Tomografievorrichtung (30), die dafür geeignet ist, um mehrere Röntgenbilder unter verschiedenen Projektionswinkeln eines Probebehälters zu erfassen, der auf einen Probenträger der Vorrichtung platziert wird;
- einen Rechner (38), der mit der Vorrichtung (39) und mit der Tomografievorrichtung (30) verbunden ist und dafür ausgelegt ist, um die Röntgenbilder zu analysieren, um:
• in einer virtuellen Markierung ein dreidimensionales digitales Modell (M) des Probebehälters aus den Röntgenbildern zu konstruieren;
• das dreidimensionale digitale Modell (M) zu analysieren, um mindestens als Qualitätsindikator (A) des Probebehälters die Darstellung von Reliefs (B) zu bestimmen, die auf dem Probebehälter angeordnet sind, wodurch ermöglicht wird, daraus eine Information der Einstellung für mindestens einen Parameter zur Steuerung des Formverfahrens in Bezug auf die Form des Probebehälters abzuleiten;
• und ein System (41) zur Bereitstellung mindestens eines Qualitätsindikators (A) des Probebehälters in Bezug auf mindestens eine Region des Probebehälters.

20. Maschine gemäß dem vorhergehenden Anspruch, **dadurch gekennzeichnet, dass** das System (41) zur Bereitstellung mindestens eines Qualitätsindikators des Probebehälters in Bezug auf mindestens eine Region des Probebehälters ein Anzeigesystem (42) für den Qualitätsindikator in Bezug auf die Identität der Endform liefert.

21. Maschine gemäß dem vorhergehenden Anspruch, **dadurch gekennzeichnet, dass** das System (41) zur Bereitstellung mindestens eines Qualitätsindikators des Probebehälters in Bezug auf mindestens eine Region des Probebehälters eine Verbindung (43) umfasst, um zu einem Steuersystem (23) der Formanlage den Qualitätsindikator (A) in Bezug auf die Identität der Endform zu übertragen.

22. Maschine gemäß einem der Ansprüche 19 bis 21, **dadurch gekennzeichnet, dass** sie ein System (40) umfasst, das an den Rechner (38) die Nummer der Form oder der Platzierung des Probebehälters (2) liefert.

23. Anlage zum Formen von Glasbehältern, umfassend mehrere verschiedene Formabschnitte (12), wobei in jedem davon mindestens ein geschmolzener Glasposten (18) zuerst aus einem Rohling in mindestens einer Vorform (13) geformt wird, dann als zweites in mindestens einer Endform (14) endgeformt wird, **dadurch gekennzeichnet, dass** sie eine Maschine (21) nach einem der Ansprüche 19 bis 22 umfasst, die am Ausgang der Endformen angeordnet ist.

## Claims

1. - A method for controlling a process for forming glass containers (**2**) implementing a facility with several separate forming sections (**12**) in each of which at least one gob of molten glass (**18**) is firstly formed into a blank in at least one preform mold (**13**), then secondly given its final forming in at least one finishing mold (**14**), **characterized in that** it includes the following steps :
- extracting a so-called sample container coming from an identified preform mold (**13**) and an identified finishing mold (**14**) ;
- resting the sample container (**2**) on a sample holder (**31**) of a computer-assisted X-ray tomography apparatus (**30**) ;
- acquiring by means of the tomography apparatus (**30**) several X-ray images of the sample container from different projection angles ;
- sending the X-ray images to a computer (**38**) ;
- analyzing X-ray images using the computer to :
• construct in a virtual reference frame a three-dimensional digital model (**M**) of the sample container on the basis of the X-ray images ;
- and analyzing the three-dimensional digital model (**M**) to determine at least as a quality indicator (**A**) of the sample container the rendering of reliefs (**B**) arranged on the sample container, making it possible to deduce from it an item of adjustment information for at least one control parameter of the forming process in relation to the mold of the sample container.

2. - The method as claimed in the preceding claim, **characterized in that** it consists in determining a quality indicator (**A**) of the sample container, making it possible to deduce an item of adjustment information for at least one control parameter of the process for forming containers for the identified molds, from among :
- the weight or the shape of the glass gob loaded into the identified preform mold ;
- the position or the speed of the glass gob (**18**) upon its loading into the identified preform mold ;
- a synchronization or speed or force in the movement of the mechanisms of the blowing plungers, of the identified molds, of the transfers of the blank, or of the extracting grippers ;
- the cooling of the identified molds or of an associated plunger;
- a blowing or pressing pressure for the identified molds ;
- the replacement of an identified mold.

3. - The method as claimed in one of the preceding claims, **characterized in that** it consists in determining as a quality indicator (**A**) of the sample container (**2**) the distribution of the glass of the sample container.

4. - The method as claimed in one of the preceding claims, **characterized in that** it consists in determining as a quality indicator (**A**) of the sample container at least one volume measurement of the sample container taken from among the capacity (**Cn**) of the sample container, the volume of the envelope of the sample container and the glass volume of the sample container.

5. - The method as claimed in one of the preceding claims, **characterized in that** it consists in determining as a quality indicator (**A**) of the sample container the internal geometry of the neck of the sample container.

6. - The method as claimed in one of the preceding claims, **characterized in that** it consists in determining as a quality indicator (**A**) of the sample container the planarity of the ring surface of the sample container.

7. - The method as claimed in one of the preceding claims, **characterized in that** it consists in determining as a quality indicator (**A**) of the sample container, a number of external diameters of the body of the sample container.

8. - The method as claimed in claim 3, **characterized in that** it consists, for determining the glass distribution as a quality indicator (**A**) of the sample container, in determining the thickness of the glass wall over at least one region of the sample container (**2**), searching in this region for the position of an area with a thickness greater than a predefined value and/or a thickness less than a predefined value, where applicable by determining the extent of said area, and/or searching for the presence and the position of the place in the wall exhibiting the minimum or the maximum thickness in said region.

9. - The method as claimed in claim 3, **characterized in that** it consists for determining the glass distribution as a quality indicator of the sample container, in :
- determining the volume of glass contained in at least two regions of the three-dimensional digital model divided either by a plane of vertical section containing the virtual vertical axis of the three-dimensional digital model or by a plane of horizontal section perpendicular to said virtual vertical axis ;
- and in comparing said volumes with values of reference volume and/or between several regions of one and the same sample container, and/or between several sample containers.

10. - The method as claimed in claim 1, **characterized in that** it consists, for determining the rendering of reliefs (**B**) fashioned on the sample container as a quality indicator of the sample container, in :
- positioning at least one section plane (**C-C**) on the three-dimensional digital model (**M**) of the sample container in such a way that it selects at least a part of a virtual relief (**Br**) of the external surface (**Se**) of said model and corresponding to the relief (**B**) ;
- determining in the section plane, the representative curve (**Cr**) of the section of the virtual relief (**Br**) ;
- overlaying at least partly on the representative curve (**Cr**), a zero-altitude curve (**Ca**) representing the curve of the external surface **(Se**) of the sample container devoid of said virtual relief (**Br**) ;
- comparing the representative curve (**Cr**) with the zero-altitude curve (**Ca**), as the criterion of rendering of the virtual relief (**Br**) at least one of the following quantities :
• a distance between the representative curve (**Cr**) and the zero-altitude curve (**Ca**) ;
• a separation in slope at a given position between the representative curve (**Cr**) and the zero-altitude curve (**Ca**) ;
• a variation in the slope of the representative curve (**Cr**) ;
• an area delimited by the representative curve (**Cr**) and the zero-altitude curve (**Ca**).

11. - The method as claimed in one of claims 1 or 10, **characterized in that** it consists, for determining the rendering of reliefs (**B**) fashioned on the sample container (**2**) as a quality indicator (**A**) of the sample container, in :
- determining the representative surface (**Sr**) of the relief as a portion of the external surface of the three-dimensional digital model in the area of interest containing at least a part of a virtual relief corresponding to the relief (**B**) ;
- overlaying at least partly on the external surface of the area of interest, a zero-altitude surface (**Sa**) representing the surface of the area of interest devoid of said virtual relief ;
- comparing the representative surface (**Sr**) with the zero-altitude surface (**Sa**), by computing as relief rendering criterion at least one of the following quantities :
• a distance between the zero-altitude surface (**Sa**) and the representative surface (**Sr**) ;
• the separation in slope at a given position between the zero-altitude surface (**Sa**) and the representative surface (**Sr**) ;
• a variation in the slopes of the representative surface (**Sr**) ;
• a number of volumes delimited by the zero-altitude surface (**Sa**) and the representative surface (**Sr**).

12. - The method as claimed in one of claims 1, 10 or 11, **characterized in that** it consists, for determining the rendering of reliefs fashioned on the sample container as a quality indicator of the sample container, in :
- determining the representative surface of a virtual relief (**Sr**) as a portion of the external surface of the three-dimensional digital model in the area of interest containing at least a part of the virtual relief corresponding to the relief of the sample container;
- overlaying at least partly on the external surface of the area of interest, a theoretical relief surface (**Sri**) representing the surface of the area of interest if the virtual relief is correctly rendered ;
- comparing the representative surface (**Sr**) with the theoretical surface (**Sri**), by computing as relief rendering criterion at least one of the following quantities :
• a distance between the representative surface (**Sr**) and the theoretical surface (**Sri**) ;
• a separation in slope at a given position between the surfaces (**Sr**) and (**Sri)** ;
• a number of volumes delimited by the surfaces (**Sr**) and (**Sri**).

13. - The method as claimed in one of claims 1, 10, 11 or 12, **characterized in that** it consists, for determining the rendering of reliefs fashioned on the sample container as a quality indicator of the sample container, in :
- selecting on the three-dimensional digital model (**M**) a virtual relief corresponding to a relief with a technical purpose, the position of which is known ;
- positioning a section plane in such a way that it cuts said relief in a section plane corresponding to a design plane ;
- obtaining a representative curve (**Cr**) of the section of the virtual relief ;
- measuring on this representative curve a radius of curvature and/or an angle, a length, or a distance to a zero-altitude curve (**Ca**) ;
- comparing the measurement with predefined tolerance values.

14. - The method as claimed in the preceding claim, **characterized in that** it consists in analyzing the three-dimensional digital model (**M**) by searching for bubbles corresponding to material lacks between the internal surface (**Sf**) and the external surface (**Se**), and in measuring the volumes of said bubbles, which are then subtracted from the volume of the wall of the three-dimensional digital model (**M**), determined between the internal surface (**Sf**) and the external surface (**Se**), with a view to obtaining a volume corresponding to the volume of glass of the gob loaded into the identified preform mold, the blank of which has been transferred into the finishing mold from which the sample container (**2**) has come.

15. - The method as claimed in the preceding claim, **characterized in that** it consists in :
- considering as being a measurement of the volume of the gob loaded into the preform mold, the volume of glass of the three-dimensional digital model (**M**), either taking the material lacks into account or not taking them into account;
- considering the internal volume delimited by the external surface of the three-dimensional digital model (**M**) and an enclosing plane as being a measurement of the internal volume of the finishing mold ;
- considering the volume delimited by the internal surface of the three-dimensional digital model (**M**) and a filling level plane as being a measurement of the capacity (**Cn**) of the sample container;
- deducing from measurements of the capacity (**Cn**) of the sample container and of the internal volume of the finishing mold, the volume of the gob to be loaded into the preform mold from which the sample container has come ;
- and deciding when the capacity of the sample container is not compliant, to modify the weight of the gob for at least the preform mold from which the sample container has come or to replace the finishing mold.

16. - The method as claimed in claim 5, **characterized in that** it consists, for determining the geometry of the neck of the sample container as a quality indicator of the sample container, in :
- determining on the three-dimensional digital model (**M**) the internal surface corresponding at least to that of the neck ;
- positioning at least one section plane (Pg) parallel to a virtual resting plane (**Pr**) ;
- measuring in this plane several diameters of the internal surface and determining the minimum and/or the maximum in the section plane;
- determing as an indicator of the neck geometry :
* the opening diameter;
* and/or the broaching diameter ;
* and/or the internal profile of the sample container.

17. - The method as claimed in claim 6, **characterized in that** it consists, for determining the planarity of the ring surface of the sample container as a quality indicator of the sample container, in :
- determining on the basis of the three-dimensional digital model (**M**), a closed three-dimensional curve or an annular surface representative of the ring surface ;
- positioning a reference plane of the ring surface in relation to the closed three-dimensional curve or to the annular surface ;
- and measuring the separations between the reference plane and the closed three-dimensional curve or the annular surface.

18. - The method as claimed in claim 7, **characterized in that** it consists, for determining the external diameters of the body of the sample container as a quality indicator of the sample container, in :
- determining on the basis of the three-dimensional digital model (**M**), the external surface (**Se**) corresponding to at least the part of the sample container for which an external diameter is to be measured ;
- positioning a section plane (**Pd**) parallel to the virtual resting plane (**Pr**) of the model along at least one height of the container;
- measuring several diameters in this section plane with respect to the external surface and comparing these measurements with reference values.

19. - A machine for controlling a process for forming glass containers implementing a facility with several separate forming sections (**12**) in each of which at least one gob of molten glass (**18**) is firstly formed into a blank in at least one preform mold (**13**), then secondly given its final form in at least one finishing mold (**14**), **characterized in that** it contains :
- a computer-assisted X-ray tomography apparatus (**30**), able to take several X-ray images from different projection angles of a sample container placed on a sample holder of said apparatus ;a computer (**38**) linked to the device (**39**) and to the tomography apparatus (**30**) and configured for analyzing the X-ray images for:
• constructing in a virtual reference frame a three-dimensional digital model (**M**) of the sample container on the basis of the X-ray images analyzing the three-dimensional digital model (**M**) to determine at least as a quality indicator (**A**) of the sample container the rendering of reliefs (**B**) arranged on the sample container, making it possible to deduce from it an item of adjustment information for at least one control parameter of the forming process in relation to the mold of the sample container;
- and a system (**41**) for delivering at least the quality indicator (**A**) of the sample container in relation to at least one region of the sample container.

20. - The machine as claimed in the preceding claim, **characterized in that** the system (**41**) for delivering at least the quality indicator of the sample container in relation to at least one region of the sample container includes a display system (**42**) for the quality indicator in relation to the identity of the finishing mold.

21. - The machine as claimed in the preceding claim, **characterized in that** the system (**41**) for delivering at least the quality indicator of the sample container in relation to at least one region of the sample container includes a connection (**43**) for sending to a control system (**23**) of the forming facility, the quality indicator (**A**) in relation to the identity of the finishing mold.

22. - The machine as claimed in one of claims 19 to 21, **characterized in that** it includes a system (**40**) supplying to the computer (**38**) the mold or location number of the sample container (**2**).

23. - A facility for forming glass containers including several separate forming sections (**12**) in each of which at least one gob of molten glass (**18**) is firstly formed into a blank in at least one preform mold (**13**), then secondly given its final form in at least one finishing mold (**14) characterized in that** it includes a machine (**21**) as claimed in one of claims 19 to 22, arranged at the exit of the finishing molds.
